(19) Europäisches Patentamt
European Patent Office
Office européen des brevets

(11) EP 2 440 174 B1

(12) # EUROPÄISCHE PATENTSCHRIFT

(45) Veröffentlichungstag und Bekanntmachung des Hinweises auf die Patenterteilung:
**17.12.2014 Patentblatt 2014/51**

(21) Anmeldenummer: **10719304.7**

(22) Anmeldetag: **27.04.2010**

(51) Int Cl.:
***A61K 8/49*** *(2006.01)* ***A61Q 5/00*** *(2006.01)*

(86) Internationale Anmeldenummer:
**PCT/EP2010/055599**

(87) Internationale Veröffentlichungsnummer:
**WO 2010/142493 (16.12.2010 Gazette 2010/50)**

(54) **NACHBEHANDLUNG ZUR FARBFIXIERUNG**

AFTERTREATMENT FOR COLOR FIXATION

POST-TRAITEMENT POUR FIXER LA COULEUR

(84) Benannte Vertragsstaaten:
**AT BE BG CH CY CZ DE DK EE ES FI FR GB GR HR HU IE IS IT LI LT LU LV MC MK MT NL NO PL PT RO SE SI SK SM TR**

(30) Priorität: **08.06.2009 DE 102009026817**

(43) Veröffentlichungstag der Anmeldung:
**18.04.2012 Patentblatt 2012/16**

(73) Patentinhaber: **Henkel AG & Co. KGaA
40589 Düsseldorf (DE)**

(72) Erfinder:
- **GROSS, Wibke
41836 Hückelhoven (DE)**
- **KNÜBEL, Georg
40219 Düsseldorf (DE)**
- **KROOS, Astrid
40789 Monheim (DE)**
- **NEMITZ, Ralph
41363 Jüchen (DE)**

(56) Entgegenhaltungen:
**WO-A2-2010/130510**
**WO-A2-2010/130513**
**WO-A2-2010/130526**
**DE-A1-102007 047 685**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 2 440 174 B1

**Beschreibung**

Die vorliegende Erfindung betrifft Mittel und Verfahren zur Nachbehandlung von bevorzugt oxidativ gefärbten Haaren, wobei durch die Nachbehandlung eine Verbesserung der Waschechtheit, insbesondere bei rötlichen Farbnuancen, erreicht wird. Die Nachbehandlungsmittel enthalten dazu ein spezielles, kationisches Acetylpyridiniumderivat. Weiterhin betrifft die vorliegende Erfindung eine Mehrkomponentenverpackungseinheit (Kit), welche Färbemittel für Haare, insbesondere ein oxidatives Färbemittel, bevorzugt für rote Nuancen, in Kombination mit einer Entwicklerzubereitung und ein Nachbehandlungsmittel umfasst.

Die Veränderung von Form und Farbe der Haare stellt einen wichtigen Bereich der modernen Kosmetik dar. Dadurch kann das Erscheinungsbild der Haare sowohl aktuellen Modeströmungen als auch den individuellen Wünschen der einzelnen Person angepasst werden. Zur Bereitstellung farbverändernder kosmetischer Mittel, insbesondere für keratinhaltige Fasern wie beispielsweise menschliche Haare, kennt der Fachmann je nach Anforderungen an die Färbung diverse Färbesysteme.

Für permanente, intensive Färbungen mit entsprechenden Echtheitseigenschaften werden sogenannte Oxidationsfärbemittel verwendet. Solche Färbemittel enthalten Oxidationsfarbstoffvorprodukte, unterteilt in sogenannte Entwicklerkomponenten und Kupplerkomponenten, die unter dem Einfluss von Oxidationsmitteln oder von Luftsauerstoff untereinander die eigentlichen Farbstoffe ausbilden. Die Oxidationsfärbemittel zeichnen sich in der Regel durch lang anhaltende Färbeergebnisse aus. Für temporäre Färbungen werden üblicherweise Färbe- oder Tönungsmittel verwendet, die als färbende Komponente sogenannte direktziehende Farbstoffe enthalten. Hierbei handelt es sich um Farbstoffmoleküle, die direkt auf das Substrat aufziehen und keinen oxidativen Prozess zur Ausbildung der Farbe benötigen. Schließlich hat ein weiteres Färbeverfahren große Beachtung gefunden, bei dem Vorstufen des natürlichen Haarfarbstoffes Melanin auf das Substrat, z. B. Haare, aufgebracht werden, wobei diese dann im Rahmen oxidativer Prozesse im Haar naturanaloge Farbstoffe ausbilden. Sollen Substrate aufgehellt oder gar gebleicht werden, werden die das Substrat färbenden Farbstoffe meist oxidativ unter Einsatz von entsprechenden Oxidationsmitteln, wie beispielsweise Wasserstoffperoxid, entfärbt.

Neben der Erzeugung von Naturnuancen, welche zumeist bei der dezenten Kaschierung von grauen Haaren Anwendung finden, ist die Ausbildung von modischen Nuancen ein Hauptanwendungsgebiet der oxidativen Haarfarben (Oxidationsfärbemittel). Insbesondere intensive Rot-, Rotbraun- und Kupfernuancen liegen bei der modischen Farbpalette im Fokus. Dem Fachmann ist bereits lange bekannt, dass jedoch die meisten Rotnuancen Schwächen in der Waschechtheit besitzen, welche sich in einer unerwünschten Verminderung der Farbintensität nach wiederholten Haarwäschen manifestieren. Ist die Haarstruktur durch Umwelteinflüsse, wie Schweiß, Sonneneinstrahlung, oder vorangegangene kosmetische Behandlungen, wie Dauerwellbehandlung oder Blondierung, geschädigt, erfolgt der durch Auswaschen bedingte Farbverlust umso schneller.

Bei leuchtenden Modetönen wird ein Verlust der Farbintensität als besonders nachteilig empfunden. Ein Mittel oder eine Methode, welche diesen bekannten Nachteil in zufriedenstellender Weise zu lösen vermag, ist bislang nicht literaturbekannt. Es ist daher die Aufgabe der vorliegenden Erfindung, ein Verfahren zur Verfügung zu stellen, mittels welchem die Farbfixierung nach einer oxidativen Färbung erhöht und die Waschechtheit bestehender Rotnuancen verbessert werden kann. Dadurch kann der Farbverlust beziehungsweise das Ausbluten der Farbe nach wiederholten Haarwäschen vermindert werden. Insbesondere die Verbesserung der Waschechtheit von Rotnuancen auf geschädigtem Haar konnte bislang nur unbefriedigend gelöst werden und stellt daher eine besondere Herausforderung für den Entwickler neuer Färbe- und Nachbehandlungssysteme dar.

Es war nicht vorhersehbar, dass die Waschstabilität von Rotnuancen durch Applikation eines Nachbehandlungsmittels, welches mindestens ein spezielles, kationisches Acetylpyridiniumderivat beinhaltet, entscheidend verbessert werden kann. Die Substanzklasse der erfindungsgemäßen Acetylpyridiniumderivate ist aus der Literatur bereits als Mittel zur Erzeugung von Färbungen auf Haaren (DE 197 45 356) oder aus Mitteln zur Aufhellung von Haaren (DE 10 2007 047685) bekannt. Aus diesen Anmeldungen ist für den Fachmann jedoch keinerlei Hinweis auf Verbesserungen im Farberhalt von oxidativen Färbungen, insbesondere von Rotnuancen, zu entnehmen.

Ein erster Gegenstand der vorliegenden Erfindung ist daher die Verwendung eines kosmetischen Nachbehandlungsmittels zur Verbesserung der Waschechtheit von auf dem Haar erzeugten Färbungen und zur Farbfixierung, dadurch gekennzeichnet, dass das Nachbehandlungsmittel in einem kosmetischen Träger mindestens ein Acetylpyridiniumderivat der Formel (I),

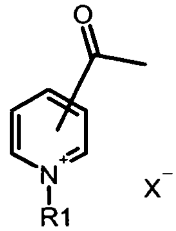

(I),

worin

R1 für eine $C_1$-$C_6$-Alkylgruppe, eine $C_2$-$C_6$-Alkenylgruppe, eine $C_2$-$C_6$-Hydroxyalkylgruppe, eine $C_1$-$C_6$-Alkoxy-$C_2$-$C_6$-alkylgruppe, eine Carboxy-$C_2$-$C_6$-alkylgruppe, eine Aryl-$C_1$-$C_6$-alkylgruppe, eine Heteroaryl-$C_1$-$C_6$-alkylgruppe, eine Arylgruppe oder eine Heteroarylgruppe und

$X^-$ für ein physiologisch verträgliches Anion stehen, enthält.

Die Nachbehandlungsmittel des ersten Erfindungsgegenstands enthalten die Wirkstoffe in einem kosmetischen Träger. Dieser kosmetische Träger ist bevorzugt wässrig, alkoholisch oder wässrigalkoholisch. Solche Träger sind beispielsweise Cremes, Emulsionen, Gele oder auch tensidhaltige schäumende Lösungen, wie beispielsweise Shampoos, Schaumaerosole oder andere Zubereitungen, die für die Anwendung auf dem Haar geeignet sind. Ein wässriger Träger enthält im Sinne der Erfindung mindestens 40 Gew.-%, insbesondere mindestens 50 Gew.-% Wasser. Unter wässrigalkoholischen Trägern sind im Sinne der vorliegenden Erfindung wasserhaltige Zusammensetzungen, enthaltend 3 bis 70 Gew.-% eines $C_1$-$C_4$-Alkohols, insbesondere Ethanol oder Isopropanol, zu verstehen. Die erfindungsgemäßen Mittel können zusätzlich weitere organische Lösungsmittel, wie beispielsweise Methoxybutanol, Ethyldiglykol, 1,2-Propylenglykol, n-Propanol, n-Butanol, n-Butylenglykol, Glycerin, Diethylenglykolmonoethylether, und Diethylenglykolmono-n-butylether, enthalten. Bevorzugt sind dabei alle wasserlöslichen organischen Lösungsmittel. Bevorzugte Mittel sind dadurch gekennzeichnet, dass sie zusätzlich ein nichtwässriges Lösungsmittel enthalten, wobei besonders bevorzugte erfindungsgemäße Mittel das Lösungsmittel in einer Konzentration von 0,1 bis 30 Gew.-%, bevorzugt in einer Konzentration von 1 bis 20 Gew.-%, ganz besonders bevorzugt in einer Konzentration von 2 bis 10 Gew.-%, jeweils bezogen auf das Mittel, enthalten.

Als wesentlichen Inhaltsstoff enthalten die erfindungsgemäß verwendeten Mittel ein Acetylpyridiniumderivat gemäß Formel (I). Im Folgenden werden Beispiele für die als Substituenten der Verbindungen der Formel (I) genannten Reste aufgezählt:

Beispiele für $C_1$-$C_6$-Alkylreste sind die Gruppen $-CH_3$, $-CH_2CH_3$, $-CH_2CH_2CH_3$, $-CH(CH_3)_2$, $-CH_2CH_2CH_2CH_3$, $-CH_2CH(CH_3)_2$, $-CH(CH_3)CH_2CH_3$, $-C(CH_3)_3$.

Beispiele für eine $C_2$-$C_6$-Alkenylgruppe sind eine Prop-2-enylgruppe (Allylgruppe), eine 2-Methyl-prop-2-enylgruppe, eine But-3-enylgruppe, eine But-2-enylgruppe, eine Pent-4-enylgruppe oder eine Pent-3-enylgruppe, wobei die Prop-2-enylgruppe bevorzugt ist.

Beispiele für eine $C_2$-$C_6$-Hydroxyalkylgruppe sind $-CH_2CH_2OH$, $-CH_2CH_2CH_2OH$, $-CH_2CH(OH)CH_3$ und $-CH_2CH_2CH_2CH_2OH$, wobei die Gruppe $-CH_2CH_2OH$ bevorzugt ist.

Beispiele für $C_1$-$C_6$-Alkoxy-$C_2$-$C_6$-alkylgruppen sind die Gruppen $-CH_2CH_2OCH_3$, $-CH_2CH_2CH_2OCH_3$, $-CH_2CH_2OCH_2CH_3$, $-CH_2CH_2CH_2OCH_2CH_3$, $-CH_2CH_2OCH(CH_3)_2$, $-CH_2CH_2CH_2OCH(CH_3)_2$.

Beispiele für eine Carboxy-$C_1$-$C_6$-alkylgruppe sind die Carboxymethylgruppe, die 2-Carboxyethylgruppe oder die 3-Carboxypropylgruppe.

Beispiele für Aryl-$C_1$-$C_6$-alkylgruppen sind die Benzylgruppe und die 2-Phenylethylgruppe.

Beispiele für eine Heteroaryl-$C_1$-$C_6$-alkylgruppe sind die Pyridin-2-ylmethylgruppe, die Pyridin-3-yl-methylgruppe, die Pyridin-4-ylmethylgruppe, die Pyrimidin-2-ylmethylgruppe, die Pyrrol-1-ylmethyl-gruppe, die Pyrrol-1-ylethylgruppe, die Pyrazol-1-ylmethylgruppe oder die Pyrazol-1-ylethylgruppe.

Beispiele für eine Arylgruppe sind die Phenylgruppe, die 1-Naphthylgruppe oder die 2-Naphthylgruppe.

Beispiele für eine Heteroarylgruppe sind die Pyridin-2-ylgruppe, die Pyridin-3-ylgruppe, die Pyridin-4-ylgruppe, die Pyrimidin-2-ylgruppe, die Pyrrol-1-ylgruppe, die Pyrrol-2-ylgruppe, die Pyrazol-1-ylgruppe, die Pyrazol-3-ylgruppe oder die Pyrazol-4-ylgruppe.

In einer Ausführungsform der vorliegenden Erfindung sind solche Verbindungen gemäß Formel (I) bevorzugt, bei welchen der Rest R1 der allgemeinen Struktur (I) für eine $C_1$-$C_6$-Alkylgruppe, für eine $C_2$-$C_6$-Alkenylgruppe oder für eine $C_2$-$C_6$-Hydroxyalkylgruppe steht.

Es ist erfindungsgemäß besonders bevorzugt, wenn der Rest R1 für eine $C_1$-$C_6$-Alkylgruppe, bevorzugt für

Methyl, Ethyl, n-Propyl oder Isopropyl und insbesondere bevorzugt für Methyl, steht.

Es ist bevorzugt, wenn das Anion $X^-$ gemäß Formel (I) ausgewählt wird aus Halogenid, insbesondere Chlorid, Bromid und Iodid, Benzolsulfonat, p-Toluolsulfonat, $C_1$-$C_4$-Alkylsulfonat, Trifluormethansulfonat, Acetat, Trifluoracetat, Perchlorat, ½ Sulfat, Hydrogensulfat, Tetrafluorborat, Hexafluorphosphat oder Tetrachlorzinkat. Erfindungsgemäß besonders begünstigt ist es, wenn das physiologisch verträgliche Anion $X^-$ für ein Halogenidion (insbesondere Chlorid oder Bromid), Hydrogensulfat, ½ Sulfat, p-Toluolsulfonat, Benzolsulfonat oder Acetat steht.

Es hat sich herausgestellt, dass die Acetylpyridiniumderivate gemäß Formel (I) erfindungsgemäß besonders vorteilhafte Eigenschaften besitzen, wenn sie die Acetyl-Gruppe entweder in 2- oder 4-Position am Pyridin-Ring tragen. Eine weitere Ausführungsform der vorliegenden Erfindung ist dadurch gekennzeichnet, dass das Mittel als Acetylpyridiniumderivat gemäß Formel (I) mindestens ein 2-Acetylpyridiniumderivat und/oder 4-Acetylpyridiniumderivat enthält.

Geeignete Acetylpyridiniumderivate sind dabei die physiologisch verträglichen Salze, die als Kation ein Acetylpyridiniumderivat, ausgewählt aus 4-Acetyl-1-methylpyridinium, 4-Acetyl-1-allyl-pyridinium, 4-Acetyl-1-(2-hydroxyethyl)pyridinium, 2-Acetyl-1-methylpyridinium, 2-Acetyl-1-allyl-pyridinium und 2-Acetyl-1-(2-hydroxyethyl)pyridinium, enthalten.

Insbesondere sind solche Mittel erfindungsgemäß geeignet, die dadurch gekennzeichnet sind, dass das Acetylpyridiniumderivat gemäß Formel (I) ausgewählt ist aus der Gruppe, die gebildet wird aus 4-Acetyl-1-methylpyridinium-p-toluolsulfonat, 4-Acetyl-1-methylpyridinium-benzolsulfonat, 4-Acetyl-1-methylpyridiniumbromid, 4-Acetyl-1-methylpyridiniumchlorid, 4-Acetyl-1-methyl-pyridiniumhydrogensulfat, 4-Acetyl-1-methylpyridiniumacetat, 4-Acetyl-1-allylpyridinium-p-toluolsulfonat, 4-Acetyl-1-allylpyridinium-benzolsulfonat, 4-Acetyl-1-allylpyridiniumbromid, 4-Acetyl-1-allylpyridiniumchlorid, 4-Acetyl-1-allylpyridiniumhydrogensulfat, 4-Acetyl-1-allylpyridiniumacetat, 2-Acetyl-1-methylpyridinium-p-toluolsulfonat, 2-Acetyl-1-methylpyridinium-benzolsulfonat, 2-Acetyl-1-methylpyridiniumbromid, 2-Acetyl-1-methylpyridiniumchlorid, 2-Acetyl-1-methylpyridinium-hydrogensulfat, 2-Acetyl-1-methylpyridiniumacetat, 2-Acetyl-1-allylpyridinium-p-toluolsuffonat, 2-Acetyl-1-allylpyridinium-benzolsulfonat, 2-Acetyl-1-allylpyridiniumbromid, 2-Acetyl-1-allylpyridiniumchlorid, 2-Acetyl-1-allylpyridiniumhydrogensulfat und/oder 2-Acetyl-1-allylpyridiniumacetat.

Dabei sind besonders vorteilhafte Nachbehandlungsmittel dadurch gekennzeichnet, dass das Acetylpyridiniumderivat gemäß Formel (I) ausgewählt ist aus 4-Acetyl-1-methylpyridinium-p-toluolsulfonat und/oder 2-Acetyl-1-methylpyridinium-p-toluolsulfonat, insbesondere 4-Acetyl-1-methylpyridinium-p-toluolsulfonat.

In einer Ausführungsform enthalten die erfindungsgemäß verwendeten Nachbehandlungsmittel die Acetylpyridiniumderivate der Formel (I) zu 0,05 bis 10 Gew.-%, bevorzugt zu 0,1 bis 7,5 Gew.-%, bevorzugt zu 0,2 bis 6,5 Gew.-% und insbesondere zu 0,5 bis 5 Gew.-%, jeweils bezogen auf das Gesamtgewicht des anwendungsbereiten Mittels.

Die Verwendung von Nachbehandlungsmitteln, enthaltend Acetylpyridiniumderivate der Formel (I), ist besonders zur Farbfixierung von oxidativen Färbungen geeignet.

Eine Ausführungsform dieses Erfindungsgegenstands ist daher dadurch gekennzeichnet, dass die Verwendung des genannten Nachbehandlungsmittels zur Verbesserung der Waschechtheit und zur Farbfixierung von oxidativen Färbungen erfolgt.

Oxidative Färbungen im Sinne der Erfindung sind Färbungen, für deren Entwicklung sogenannte Oxidationsfärbemittel verwendet wurden. Solche Färbemittel enthalten als farbverändemde Komponente üblicherweise Oxidationsfarbstoffvorprodukte, sogenannte Entwicklerkomponenten und Kupplerkomponenten, die unter dem Einfluss von Oxidationsmitteln oder von Luftsauerstoff untereinander die eigentlichen Farbstoffe ausbilden.

Die Oxidationsfarbstoffvorprodukte werden bevorzugt in einer Menge von 0,005 bis 25 Gew.-%, bevorzugt von 0,05 bis 20 Gew.-% und besonders bevorzugt von 0,1 bis 15 Gew.-%, jeweils bezogen auf das anwendungsbereite Oxidationsfärbemittel, verwendet.

Vorzugsweise enthalten die oxidativen Färbemittel mindestens ein Oxidationsfarbstoffvorprodukt vom Entwicklertyp und mindestens ein Oxidationsfarbstoffvorprodukt vom Kupplertyp.

Die Entwickler- und Kupplerkomponenten werden üblicherweise in freier Form eingesetzt. Bei Substanzen mit Aminogruppen kann es aber bevorzugt sein, sie in Salzform, insbesondere in Form der Hydrochloride und Hydrobromide oder der Sulfate einzusetzen.

Erfindungsgemäße Entwicklerkomponenten werden ausgewählt aus p-Phenylendiaminen, zweikernigen Entwicklerkomponenten, p-Aminophenolen, o-Aminophenolen, heterocyclischen Entwicklerkomponenten und/oder den Derivaten vorstehender Substanzklassen. Die Entwicklerkomponenten werden bevorzugt in einer Menge von 0,005 bis 20 Gew.-%, vorzugsweise 0,1 bis 15 Gew.-%, jeweils bezogen auf das anwendungsbereite Oxidationsfärbemittel, verwendet.

Bevorzugte p-Phenylendiamine werden ausgewählt aus einer oder mehrerer Verbindungen der Gruppe, die gebildet wird, aus p-Phenylendiamin, p-Toluylendiamin, 2-Chlor-p-phenylendiamin, 2,3-Dimethyl-p-phenylendiamin, 2,6-Dimethyl-p-phenylendiamin, 2,6-Diethyl-p-phenylendiamin, 2,5-Dimethyl-p-phenylendiamin, N,N-Dimethyl-p-phenylendiamin, N,N-Diethyl-p-phenylendiamin, N,N-Dipropyl-p-phenylendiamin, 4-Amino-3-methyl-(N,N-diethyl)anilin, N,N-Bis-(2-hydroxyethyl)-p-phenylendiamin, 4-N,N-Bis-(2-hydroxyethyl)amino-2-methylanilin, 4-N,N-Bis-(2-hydroxyethyl)-amino-2-chloranilin, 2-(2-Hydroxyethyl)-p-phenylendiamin, 2-(1,2-Dihydroxyethyl)-p-phenylen-diamin, 2-Fluor-p-phenylendiamin, 2-Isopropyl-p-phenylendiamin, N-(2-Hydroxypropyl)-p-phenylendiamin, 2-Hydroxymethyl-p-phenylendiamin, N,N-Dimethyl-3-methyl-p-phenylendiamin, N-Ethyl-N-2-hydroxyethyl-p-phenylendiamin, N-(2,3-Dihydroxypropyl)-p-phenylendiamin, N-(4'-Aminophenyl)-p-phenylendiamin, N-Phenyl-p-phenylendiamin, 2-(2-Hydroxyethyloxy)-p-phenylendiamin, 2-Methoxymethyl-p-phenylendiamin, 2-(2-Acetylaminoethyloxy)-p-phenylendiamin, N-(2-Methoxyethyl)-p-phenylendiamin, N-(4-Amino-3-methylphenyl)-N-[3-(1H-imidazol-1-yl)propyl]amin, 5,8-Diaminobenzo-1,4-dioxan sowie ihren physiologisch verträglichen Salzen. Erfindungsgemäß besonders bevorzugte p-Phenylendiaminderivate sind ausgewählt aus mindestens einer Verbindung der Gruppe p-Phenylendiamin, p-Toluylendiamin, 2-(2-Hydroxyethyl)-p-phenylendiamin, 2-(1,2-Dihydroxyethyl)-p-phenylendiamin, N,N-Bis-(2-hydroxyethyl)-p-phenylendiamin, N-(4-Amino-3-methylphenyl)-N-[3-(1H-imidazol-1-yl)propyl]amin, 2-Methoxymethyl-p-phenylendiamin sowie den physiologisch verträglichen Salzen dieser Verbindungen.

Es kann erfindungsgemäß weiterhin bevorzugt sein, als Entwicklerkomponente Verbindungen einzusetzen, die mindestens zwei aromatische Kerne enthalten, die mit Amino- und/oder Hydroxylgruppen substituiert sind. Bevorzugte zweikernige Entwicklerkomponenten werden insbesondere aus mindestens einer der folgenden Verbindungen ausgewählt: N,N'-Bis-(2-hydroxyethyl)-N,N'-bis-(4'-aminophenyl)-1,3-diamino-propan-2-ol, N,N'-Bis-(2-hydroxyethyl)-N,N'-bis-(4'-aminophenyl)ethylendiamin, N,N'-Bis-(4'-aminophenyl)tetramethylendiamin, N,N'-Bis-(2-hydroxyethyl)-N,N'-bis-(4'-aminophenyl)tetramethylendiamin, N,N'-Bis-[4-(methylamino)phenyl]-tetramethylendiamin, N,N'-Diethyl-N,N'-bis-(4'-amino-3'-methylphenyl)ethylendiamin, Bis-(2-hydroxy-5-aminophenyl)methan, N,N'-Bis-(4'-aminophenyl)-1,4-diazacycloheptan, N,N'-Bis-(2-hydroxy-5-aminobenzyl)piperazin, N-(4'-Aminophenyl)-p-phenylendiamin und 1,10-Bis-(2',5'-di-aminophenyl)-1,4,7,10-tetraoxadecan sowie ihre physiologisch verträglichen Salze. Besonders bevorzugte zweikernige Entwicklerkomponenten werden ausgewählt unter N,N'-Bis-(2-hydroxy-ethyl)-N,N'-bis-(4-aminophenyl)-1,3-diamino-propan-2-ol, Bis-(2-hydroxy-5-aminophenyl)methan, 1,3-Bis-(2,5-diaminophenoxy)propan-2-ol, N,N'-Bis-(4-aminophenyl)-1,4-diazacycloheptan, 1,10-Bis-(2,5-diaminophenyl)-1,4,7,10-tetraoxadecan oder einem der physiologisch verträglichen Salze dieser Verbindungen.

Weiterhin kann es erfindungsgemäß bevorzugt sein, als Entwicklerkomponente ein p-Amino-phenolderivat oder eines seiner physiologisch verträglichen Salze einzusetzen. Bevorzugte p-Aminophenole sind insbesondere p-Aminophenol, N-Methyl-p-aminophenol, 4-Amino-3-methyl-phenol, 4-Amino-3-fluorphenol, 2-Hydroxymethylamino-4-aminophenol, 4-Amino-3-hydroxymethyl-phenol, 4-Amino-2-(2-hydroxyethoxy)phenol, 4-Amino-2-methylphenol, 4-Amino-2-hydroxy-methylphenol, 4-Amino-2-methoxymethylphenol, 4-Amino-2-aminomethylphenol, 4-Amino-2-[(2-hydroxyethyl)aminomethyl]phenol, 4-Amino-2-(1,2-dihydroxyethyl)phenol, 4-Amino-2-fluorphenol, 4-Amino-2-chlorphenol, 4-Amino-2,6-dichlorphenol, 4-Amino-2-(diethylaminomethyl)phenol sowie ihre physiologisch verträglichen Salze. Besonders bevorzugte Verbindungen sind p-Aminophenol, 4-Amino-3-methylphenol, 4-Amino-2-aminomethylphenol, 4-Amino-2-(1,2-dihydroxyethyl)phenol und 4-Amino-2-(diethylaminomethyl)phenol oder einem der physiologisch verträglichen Salze dieser Verbindungen.

Ferner kann die Entwicklerkomponente ausgewählt sein aus o-Aminophenol und seinen Derivaten, wie beispielsweise 2-Amino-4-methylphenol, 2-Amino-5-methylphenol oder 2-Amino-4-chlorphenol.

Weiterhin kann die Entwicklerkomponente ausgewählt sein aus heterocyclischen Entwicklerkomponenten, wie beispielsweise aus Pyrimidinderivaten, Pyrazolderivaten, Pyrazolopyrimidin-Derivaten bzw. ihren physiologisch verträglichen Salzen. Bevorzugte Pyrimidinderivate sind die Verbindungen 2,4,5,6-Tetraaminopyrimidin, 4-Hydroxy-2,5,6-triaminopyrimidin, 2-Hydroxy-4,5,6-triaminopyrimidin, 2-Dimethylamino-4,5,6-triaminopyrimidin, 2,4-Dihydroxy-5,6-diaminopyrimidin und 2,5,6-Triaminopyrimidin. Bevorzugte Pyrazolderivate sind die Verbindungen, die ausgewählt werden unter 4,5-Diamino-1-methylpyrazol, 4,5-Diamino-1-(2-hydroxyethyl)pyrazol, 3,4-Diamino-pyrazol, 4,5-Diamino-1-(4'-chlorbenzyl)pyrazol, 4,5-Diamino-1,3-dimethylpyrazol, 4,5-Diamino-3-methyl-1-phenylpyrazol, 4,5-Diamino-1-methyl-3-phenylpyrazol, 4-Amino-1,3-dimethyl-5-hydrazinopyrazol, 1-Benzyl-4,5-diamino-3-methylpyrazol, 4,5-Diamino-3-t-butyl-1-methylpyrazol, 4,5-Diamino-1-t-butyl-3-methylpyrazol, 4,5-Diamino-1-(2-hydroxyethyl)-3-methylpyrazol, 4,5-Diamino-1-ethyl-3-methylpyrazol, 4,5-Diamino-1-ethyl-3-(4'-methoxyphenyl)pyrazol, 4,5-Diamino-1-ethyl-3-hydroxymethylpyrazol, 4,5-Diamino-3-hydroxymethyl-1-methylpyrazol, 4,5-Diamino-3-hydroxymethyl-1-isopropylpyrazol, 4,5-Diamino-3-methyl-1-isopropylpyrazol, 4-Amino-5-(2-aminoethyl)-amino-1,3-dimethylpyrazol, sowie deren physiologisch verträglichen Salze.

Bevorzugte Pyrazolopyrimidinderivate sind insbesondere die Derivate des Pyrazolo[1,5-a]pyrimidin und dessen tautomeren Formen, sofern ein tautomeres Gleichgewicht besteht. Die Pyrazolo[1,5-a]pyrimidinen sind insbesondere ausgewählt aus Pyrazolo[1,5-a]pyrimidin-3,7-diamin, 2,5-Dimethyl-pyrazolo[1,5-a]pyrimidin-3,7-diamin, Pyrazolo[1,5-a]pyrimidin-3,5-diamin, 2,7-Dimethylpyrazolo[1,5-a]pyrimidin-3,5-diamin, 3-Aminopyrazolo[1,5-a]pyrimidin-7-ol, 3-Aminopyrazolo[1,5-a]pyrimidin-5-ol, 2-(3-Aminopyrazolo[1,5-a]pyrimidin-7-ylamino)ethanol, 2-(7-Aminopyrazolo[1,5-a]pyrimidin-3-ylamino)ethanol, 2-[(3-Aminopyrazolo[1,5-a]pyrimidin-7-yl)-(2-hydroxyethyl)amino]-ethanol, 2-[(7-Aminopyrazolo[1,5-a]pyrimidin-3-yl)-(2-hydroxyethyl)amino]ethanol; 5,6-Dimethylpyrazolo[1,5-a]pyrimidin-3,7-diamin, 2,6-Dimethylpyrazolo[1,5-a]pyrimidin-3,7-diamin, 3-Amino-7-dimethylamino-2,5-dimethylpyrazolo[1,5-a]pyrimidin sowie ihren physiologisch verträglichen Salzen und ihren tautomeren Formen, wenn ein tautomeres Gleichgewicht vorhanden ist.

Ganz besonders bevorzugte Entwicklerkomponenten werden ausgewählt, aus mindestens einer Verbindung

aus der Gruppe, die gebildet wird aus p-Phenylendiamin, p-Toluylendiamin, 2-(2-Hydroxyethyl)-p-phenylendiamin, 2-(1,2-Dihydroxyethyl)-p-phenylendiamin, N,N-Bis-(2-hydroxyethyl)-p-phenylendiamin, 2-Methoxymethyl-p-phenylendiamin, N-(4-Amino-3-methylphenyl)-N-[3-(1 H-imidazol-1-yl)propyl]amin, N,N'-Bis-(2-hydroxyethyl)-N,N'-bis-(4-aminophenyl)-1,3-diaminopropan-2-ol, Bis-(2-hydroxy-5-aminophenyl)methan, 1,3-Bis-(2,5-diaminophenoxy)propan-2-ol, N,N'-Bis-(4-aminophenyl)-1,4-diazacycloheptan, 1,10-Bis-(2,5-diaminophenyl)-1,4,7,10-tetraoxadecan, p-Aminophenol, 4-Amino-3-methylphenol, 4-Amino-2-aminomethylphenol, 4-Amino-2-(1,2-dihydroxyethyl)phenol und 4-Amino-2-(diethylaminomethyl)phenol, 4,5-Diamino-1-(2-hydroxyethyl)-pyrazol, 2,4,5,6-Tetraaminopyrimidin, 4-Hydroxy-2,5,6-triaminopyrimidin, 2-Hydroxy-4,5,6-triaminopyrimidin, sowie deren physiologisch verträglichen Salzen.

Kupplerkomponenten bilden im Rahmen der oxidativen Färbung allein keine signifikante Färbung aus, sondem benötigen stets die Gegenwart von Entwicklerkomponenten. Daher ist es erfindungsgemäß bevorzugt, dass bei Verwendung mindestens einer Kupplerkomponente zusätzlich mindestens eine Entwicklerkomponente zum Einsatz kommt.

Erfindungsgemäße Kupplerkomponenten werden bevorzugt ausgewählt aus m-Aminophenol, m-Diaminobenzol, o-Diaminobenzol, o-Aminophenol, Naphthalinderivaten mit mindestens einer Hydroxygruppe, Di- beziehungsweise Trihydroxybenzol, Pyridin, Pyrimidin, Monohydroxy- bzw. Monoaminoindol, Monohydroxy- bzw. Monoaminoindolin, Pyrazolon, Benzomorpholin, Chinoxalin und/oder den Derivaten der vorstehenden Substanzklassen. Die Kupplerkomponenten werden bevorzugt in einer Menge von 0,005 bis 20 Gew.-%, vorzugsweise 0,1 bis 15 Gew.-%, jeweils bezogen auf das anwendungsbereite Oxidationsfärbemittel, verwendet.

Die erfindungsgemäß bevorzugten m-Aminophenole bzw. deren Derivate werden ausgewählt aus mindestens einer Verbindung aus der Gruppe, die gebildet wird aus m-Aminophenol, 5-Amino-2-methylphenol, N-Cyclopentyl-3-aminophenol, 3-Amino-2-chlor-6-methylphenol, 2-Hydroxy-4-aminophenoxyethanol, 2,6-Dimethyl-3-aminophenol, 3-Trifluoroacetylamino-2-chlor-6-methylphenol, 5-Amino-4-chlor-2-methylphenol, 5-Amino-4-methoxy-2-methylphenol, 5-(2'-Hydroxyethyl)amino-2-methylphenol, 3-Diethylaminophenol, N-Cyclopentyl-3-aminophenol, 1,3-Dihydroxy-5-methylaminobenzol, 3-Ethylamino-4-methylphenol, 2,4-Dichlor-3-aminophenol und den physiologisch verträglichen Salzen der vorstehend genannten Verbindungen.

Die erfindungsgemäß bevorzugten m-Diaminobenzole bzw. deren Derivate werden ausgewählt aus mindestens einer Verbindung aus der Gruppe, die gebildet wird aus m-Phenylendiamin, 2-(2,4-Diaminophenoxy)ethanol, 1,3-Bis(2,4-diaminophenoxy)propan, 1-Methoxy-2-amino-4-(2'-hydroxyethylamino)benzol, 1,3-Bis(2,4-diaminophenyl)propan, 2,6-Bis(2'-hydroxyethylamino)-1-methylbenzol, 2-({3-[(2-Hydroxyethyl)amino]-4-methoxy-5-methylphenyl}amino)ethanol, 2-({3-[(2-Hydroxyethyl)mino]-2-methoxy-5-methylphenyl}amino)ethanol, 2-({3-[(2-Hydroxyethyl)amino]-4,5-dimethylphenyl}amino)ethanol, 2-[3-Morpholin-4-ylphenyl)amino]ethanol, 3-Amino-4-(2-methoxyethoxy)-5-methylphenylamin, 1-Amino-3-bis-(2'-hydroxyethyl)aminobenzol und deren physiologisch verträglichen Salzen.

Die erfindungsgemäß bevorzugten o-Diaminobenzole bzw. deren Derivate werden ausgewählt aus mindestens einer Verbindung aus der Gruppe, die gebildet wird aus 3,4-Diaminobenzoesäure und 2,3-Diamino-1-methylbenzol und deren physiologisch verträglichen Salzen.

Bevorzugte Di- beziehungsweise Trihydroxybenzole und deren Derivate werden ausgewählt aus mindestens einer Verbindung der Gruppe, die gebildet wird aus Resorcin, Resorcinmonomethylether, 2-Methylresorcin, 5-Methylresorcin, 2,5-Dimethylresorcin, 2-Chlorresorcin, 4-Chlorresorcin, Pyrogallol und 1,2,4-Trihydroxybenzol.

Die erfindungsgemäß bevorzugten Pyridinderivate werden ausgewählt aus mindestens einer Verbindung der Gruppe, die gebildet wird aus 2,6-Dihydroxypyridin, 2-Amino-3-hydroxypyridin, 2-Amino-5-chlor-3-hydroxypyridin, 3-Amino-2-methylamino-6-methoxypyridin, 2,6-Dihydroxy-3,4-dimethylpyridin, 2,6-Dihydroxy-4-methylpyridin, 2,6-Diaminopyridin, 2,3-Diamino-6-methoxypyridin, 3,5-Diamino-2,6-dimethoxypyridin, 3,4-Diaminopyridin, 2-(2-Methoxyethyl)amino-3-amino-6-methoxypyridin, 2-(4'-Methoxyphenyl)amino-3-aminopyridin, und den physiologisch verträglichen Salzen der vorgenannten Verbindungen.

Bevorzugte Naphthalinderivate mit mindestens einer Hydroxygruppe werden ausgewählt aus mindestens einer Verbindung der Gruppe, die gebildet wird aus 1-Naphthol, 2-Methyl-1-naphthol, 2-Hydroxymethyl-1-naphthol, 2-Hydroxyethyl-1-naphthol, 1,3-Dihydroxynaphthalin, 1,5-Dihydroxynaphthalin, 1,6-Dihydroxynaphthalin, 1,7-Dihydroxynaphthalin, 1,8-Dihydroxynaphthalin, 2,7-Dihydroxynaphthalin und 2,3-Dihydroxynaphthalin.

Die erfindungsgemäß bevorzugten Indolderivate werden ausgewählt aus mindestens einer Verbindung der Gruppe, die gebildet wird aus 4-Hydroxyindol, 6-Hydroxyindol und 7-Hydroxyindol und den physiologisch verträglichen Salzen der vorgenannten Verbindungen.

Die erfindungsgemäß bevorzugten Indolinderivate werden ausgewählt aus mindestens einer Verbindung der Gruppe, die gebildet wird aus 4-Hydroxyindolin, 6-Hydroxyindolin und 7-Hydroxy-indolin und den physiologisch verträglichen Salzen der vorgenannten Verbindungen.

Bevorzugte Pyrimidinderivate werden ausgewählt aus mindestens einer Verbindung der Gruppe, die gebildet wird aus 4,6-Diaminopyrimidin, 4-Amino-2,6-dihydroxypyrimidin, 2,4-Diamino-6-hydroxypyrimidin, 2,4,6-Trihydroxypyrimidin, 2-Amino-4-methylpyrimidin, 2-Amino-4-hydroxy-6-methylpyrimidin und 4,6-Dihydroxy-2-methylpyrimidin und den physiologisch verträglichen Salzen der vorgenannten Verbindungen.

Erfindungsgemäß besonders bevorzugte Kupplerkomponenten werden ausgewählt unter 3-Aminophenol, 5-Amino-2-methylphenol, 3-Amino-2-chlor-6-methylphenol, 2-Hydroxy-4-amino-phenoxyethanol, 5-Amino-4-chlor-2-methylphenol, 5-(2-Hydroxyethyl)-amino-2-methylphenol, 2,4-Dichlor-3-aminophenol, 2-Aminophenol, 3-Phenylendiamin, 2-(2,4-Diaminophenoxy)ethanol, 1,3-Bis-(2,4-diaminophenoxy)propan, 1-Methoxy-2-amino-4-(2-hydroxyethylamino)benzol, 1,3-Bis-(2,4-diaminophenyl)propan, 2,6-Bis-(2'-hydroxyethylamino)-1-methylbenzol, 2-({3-[(2-Hydroxyethyl)-amino]-4-methoxy-5-methylphenyl}amino)ethanol, 2-({3-[(2-Hydroxyethyl)amino]-2-methoxy-5-methylphenyl}amino)ethanol, 2-({3-[(2-Hydroxyethyl)amino]-4,5-dimethylphenyl}amino)ethanol, 2-[3-Morpholin-4-ylphenyl)amino]ethanol, 3-Amino-4-(2-methoxyethoxy)-5-methylphenylamin, 1-Amino-3-bis-(2-hydroxyethyl)aminobenzol, Resorcin, 2-Methylresorcin, 4-Chlorresorcin, 1,2,4-Trihydroxybenzol, 2-Amino-3-hydroxypyridin, 3-Amino-2-methylamino-6-methoxypyridin, 2,6-Dihydroxy-3,4-dimethylpyridin, 3,5-Diamino-2,6-dimethoxypyridin, 1-Phenyl-3-methylpyrazol-5-on, 1-Naphthol, 1,5-Dihydroxynaphthalin, 2,7-Dihydroxynaphthalin, 1,7-Dihydroxynaphthalin, 1,8-Dihydroxynaphthalin, 4-Hydroxyindol, 6-Hydroxyindol, 7-Hydroxyindol, 4-Hydroxyindolin, 6-Hydroxy-indolin, 7-Hydroxyindolin oder Gemischen dieser Verbindungen oder den physiologisch verträglichen Salzen der vorgenannten Verbindungen.

Dabei werden Entwicklerkomponenten und Kupplerkomponenten im Allgemeinen in etwa molaren Mengen zueinander eingesetzt. Wenn sich auch der molare Einsatz als zweckmäßig erwiesen hat, so ist ein gewisser Überschuss einzelner Oxidationsfarbstoffvorprodukte nicht nachteilig, so dass Entwicklerkomponenten und Kupplerkomponenten in einem Mol-Verhältnis von 3 zu 1 bis 1 zu 3, insbesondere 2 zu 1 bis 1 zu 2, stehen können.

Die erfindungsgemäße Verwendung von Nachbehandlungsmitteln, enthaltend in einem kosmetischen Träger mindestens ein Acetylpyridiniumderivat der Formel (I), hat sich als besonders vorteilhaft erwiesen, wenn es sich bei der oxidativen Färbung um eine rote, rötliche oder rotbraune Nuance handelt. Solche Nuancen lassen sich bevorzugt mit bestimmten Oxidationsfarbstoffvorprodukten erzeugen. Dazu zählen insbesondere heterocyclische Oxidationsfarbstoffvorprodukte vom Entwicklertyp.

Eine weitere Ausführungsform des ersten Erfindungsgegenstands ist daher dadurch gekennzeichnet, dass das erfindungsgemäß verwendete Nachbehandlungsmittel zur Verbesserung der Waschechtheit von oxidativen Färbungen eingesetzt wird, zu deren Ausbildung der Einsatz von mindestens einem Oxidationsfarbstoffvorprodukt vom Entwicklertyp, ausgewählt aus der Gruppe, die gebildet wird aus 2,4,5,6-Tetraaminopyrimidin, 4-Hydroxy-2,5,6-triaminopyrimidin, 2-Hydroxy-4,5,6-triaminopyrimidin, 2-Dimethylamino-4,5,6-triaminopyrimidin, 2,4-Dihydroxy-5,6-diaminopyrimidin und 2,5,6-Triaminopyrimidin sowie 4,5-Diamino-1-methylpyrazol, 4,5-Diamino-1-(2-hydroxyethyl)pyrazol, 3,4-Diaminopyrazol, 4,5-Diamino-1-(4'-chlorbenzyl)pyrazol, 4,5-Diamino-1,3-dimethylpyrazol, 4,5-Diamino-3-methyl-1-phenylpyrazol, 4,5-Diamino-1-methyl-3-phenylpyrazol, 4-Amino-1,3-dimethyl-5-hydrazinopyrazol, 1-Benzyl-4,5-diamino-3-methylpyrazol, 4,5-Diamino-3-t-butyl-1-methylpyrazol, 4,5-Diamino-1-t-butyl-3-methylpyrazol, 4,5-Diamino-1-(2-hydroxyethyl)-3-methylpyrazol, 4,5-Diamino-1-ethyl-3-methylpyrazol, 4,5-Diamino-1-ethyl-3-(4-methoxyphenyl)pyrazol, 4,5-Diamino-1-ethyl-3-hydroxymethylpyrazol, 4,5-Diamino-3-hydroxymethyl-1-methylpyrazol, 4,5-Diamino-3-hydroxymethyl-1-isopropylpyrazol, 4,5-Diamino-3-methyl-1-isopropylpyrazol, 4-Amino-5-(2-aminoethyl)amino-1,3-dimethylpyrazol, sowie deren physiologisch verträglichen Salze, zugrunde liegt.

Besonders bevorzugt wurde die oxidative Färbung unter Einsatz von mindestens einem Oxidationsfarbstoffvorprodukt vom Entwicklertyp, ausgewählt aus 4,5-Diamino-1-(2-hydroxyethyl)-pyrazol und/oder 2,4,5,6-Tetraaminopyrimidin, erzeugt.

Rote, rötliche oder rotbraune Nuancen lassen sich bevorzugt mit speziellen Kombinationen aus bestimmten Oxidationsfarbstoffvorprodukten vom Entwicklertyp und vom Kupplertyp generieren. Erfindungsgemäß bevorzugt werden die Nachbehandlungsmittel, enthaltend in einem kosmetischen Träger mindestens ein Acetylpyridiniumderivat der Formel (I), zur Verbesserung der Waschechtheit und Farbfixierung oxidativer Färbungen verwendet, wenn zur Ausbildung der Färbung mindestens eine Entwickler/Kuppler-Kombinationen eingesetzt wurde, die ausgewählt ist aus

2,4,5,6-Tetraaminopyrimidin und Resorcin;
2,4,5,6-Tetraaminopyrimidin und 2-Methylresorcin;
2,4,5,6-Tetraaminopyrimidin und 4-Chlorresorcin;
2,4,5,6-Tetraaminopyrimidin und 2,6-Bis(2'-hydroxyethylamino)-1-methylbenzol;
4,5-Diamino-1-(2-hydroxyethyl)pyrazol und Resorcin;
4,5-Diamino-1-(2-hydroxyethyl)pyrazol und 2-Methylresorcin;
4,5-Diamino-1-(2-hydroxyethyl)pyrazol und 4-Chlorresorcin;
4,5-Diamino-1-(2-hydroxyethyl)pyrazol und 3-Aminophenol;
4,5-Diamino-1-(2-hydroxyethyl)pyrazol und 3-Amino-2-chlor-6-methylphenol;
4,5-Diamino-1-(2-hydroxyethyl)pyrazol und 5-Amino-2-methylphenol;
4,5-Diamino-1-(2-hydroxyethyl)pyrazol und 1-Hydroxynaphthalin; sowie
4,5-Diamino-1-(2-hydroxyethyl)pyrazol und 2,4-Dichlor-m-aminophenol.

Im Falle der oxidativen Färbungen kann die Entwicklung der Farbe aus den Oxidationsfarbstoffvorprodukten grundsätzlich mit Luftsauerstoff erfolgen. Bevorzugt wird jedoch ein chemisches Oxidationsmittel eingesetzt, besonders dann, wenn neben der Färbung ein Aufhelleffekt an menschlichem Haar gewünscht ist. Dieser Aufhelleffekt kann unabhängig von der Färbemethode gewünscht sein. Als Oxidationsmittel kommen Persulfate, Peroxodisutfate, Chlorite,

Hypochlorite und insbesondere Wasserstoffperoxid oder und/oder eines seiner festen Anlagerungsprodukte an organische oder anorganische Verbindungen in Frage.

Um eine vorzeitige, unerwünschte Reaktion der Oxidationsfarbstoffvorprodukte durch das Oxidationsmittel zu verhindern, werden Oxidationsfarbstoffvorprodukte und Oxidationsmittel selbst zweckmäßigerweise getrennt voneinander konfektioniert und erst unmittelbar vor der Anwendung in Kontakt gebracht.

Bevorzugt enthält die Oxidationsmittelzubereitung als Oxidationsmittel Wasserstoffperoxid und/oder eines seiner festen Anlagerungsprodukte an organische oder anorganische Verbindungen, wie Harnstoff, Melamin sowie Natriumborat.

Bevorzugt beträgt die Menge an Oxidationsmittel im anwendungsbereiten Mittel 0,5 bis 12 Gew.-%, bevorzugt 2 bis 10 Gew.-% insbesondere bevorzugt zu 3 bis 6 Gew.-% (berechnet als 100 %-iges $H_2O_2$), jeweils bezogen auf das anwendungsbereite Mittel.

Solche Oxidationsmittelzubereitungen sind vorzugsweise wässrige, fließfähige Oxidationsmittelzubereitungen. Dabei sind bevorzugte Zubereitungen dadurch gekennzeichnet, dass die fließfähige Oxidationsmittelzubereitung - bezogen auf ihr Gewicht - 40 bis 90 Gew.-%, vorzugsweise 50 bis 85 Gew.-%, besonders bevorzugt 55 bis 80 Gew.-%, weiter bevorzugt 60 bis 77,5 Gew.-% und insbesondere 65 bis 75 Gew.-% Wasser enthält.

Das Oxidationsfärbemittel kann aber auch zusammen mit einem Katalysator auf das Haar aufgebracht werden, der die Oxidation der Farbstoffvorprodukte, z.B. durch Luftsauerstoff, aktiviert. Solche Katalysatoren sind z. B. bestimmte Enzyme, lodide, Chinone oder Metallionen.

Weiterhin hat es sich als vorteilhaft erweisen, wenn die Oxidationsmittelzubereitungen mindestens einen Stabilisator oder Komplexbildner enthalten. Besonders bevorzugte Stabilisatoren sind Phenacetin, Alkalibenzoate (Natriumbenzoat) und Salicylsäure. Weiterhin können alle Komplexbildner des Standes der Technik eingesetzt werden. Diese können unterschiedlichen chemischen Gruppen angehören. Vorzugsweise werden einzeln oder im Gemisch miteinander eingesetzt. Erfindungsgemäß bevorzugte Komplexbildner sind stickstoffhaltigen Polycarbonsäuren, insbesondere EDTA, und Phosphonate, vorzugsweise Hydroxyalkan- bzw. Aminoalkanphosphonate und insbesondere 1-Hydroxyethan-1,1-diphosphonat (HEDP) bzw. dessen Di- oder Tetranatriumsalz und/oder Ethylendiamintetramethylenphosphonat (EDTMP) bzw. dessen Hexanatriumsalz und/oder Diethylentriaminpentamethylenphosphonat (DTPMP) bzw. dessen Hepta- oder Octanatriumsalz.

Die anwendungsbereite Färbezubereitung sowie das Nachbehandlungsmittel können weitere Hilfs- und Zusatzstoffe enthalten. So hat es sich als vorteilhaft erwiesen, wenn die Färbezubereitung, die Oxidationsmittelzubereitung und/oder das Nachbehandlungsmittel mindestens ein Verdickungsmittel enthält. Bezüglich dieser Verdickungsmittel bestehen keine prinzipiellen Einschränkungen. Es können sowohl organische als auch rein anorganische Verdickungsmittel zum Einsatz kommen.

Geeignete Verdickungsmittel sind

- anionische, synthetische Polymere;
- kationische, synthetische Polymere;
- natürlich vorkommende Verdickungsmittel, wie nichtionische Guargums, Skleroglucangums oder Xanthangums, Gummi arabicum, Ghatti-Gummi, Karaya-Gummi, Tragant-Gummi, Carrageen-Gummi, Agar-Agar, Johannisbrotkernmehl, Pektine, Alginate, Stärke-Fraktionen und Derivate wie Amylose, Amylopektin und Dextrine, sowie Cellulosederivate, wie beispielsweise Methylcellulose, Carboxyalkylcellulosen und Hydroxyalkylcellulosen;
- nichtionische, vollsynthetische Polymere, wie Polyvinylalkohol oder Polyvinylpyrrolidinon; sowie
- anorganische Verdickungsmittel, insbesondere Schichtsilikate wie beispielsweise Bentonit, besonders Smektite, wie Montmorillonit oder Hectorit.

Zur weiteren Steigerung der Leistung der Färbemittelzubereitung können der erfindungsgemäßen Zusammensetzung zusätzlich mindestens eine $SiO_2$-Verbindung, wie Kieselsäure oder Silicate, insbesondere Wassergläser, zugesetzt. Es kann erfindungsgemäß bevorzugt sein, die $SiO_2$-Verbindungen in Mengen von 0,05 Gew.-% bis 15 Gew.-%, besonders bevorzugt in Mengen von 0,15 Gew.-% bis 10 Gew.-% und ganz besonders bevorzugt in Mengen von 0,2 Gew.-% bis 5 Gew.-%, jeweils bezogen auf die erfindungsgemäße wasserfreie Zusammensetzung, einzusetzen. Die Mengenangaben geben dabei jeweils den Gehalt der $SiO_2$-Verbindungen (ohne deren Wasseranteil) in den Mitteln wieder.

Weiterhin dient die Verwendung des genannten Nachbehandlungsmittels zur Verbesserung der Waschechtheit und zur Farbfixierung von Färbungen, die von direktziehenden Farbstoffen herrühren. Ebenso können die oxidativen Färbemittel zusätzlich mindestens einen direktziehenden Farbstoff enthalten. Dabei handelt es sich um Farbstoffe, die direkt auf das Haar aufziehen und keinen oxidativen Prozess zur Ausbildung der Farbe benötigen. Direktziehende Farbstoffe sind üblicherweise Nitrophenylendiamine, Nitroaminophenole, Azofarbstoffe, Anthrachinone oder Indophenole. Direktziehende Farbstoffe sind als anionische, kationische und nichtionische direktziehende Farbstoffe bekannt. Die direktziehenden Farbstoffe werden jeweils bevorzugt in einer Menge von 0,001 bis 20 Gew.-%, bezogen auf die

gesamte Anwendungszubereitung, eingesetzt.

Bevorzugte anionische direktziehende Farbstoffe sind die unter den internationalen Bezeichnungen bzw. Handelsnamen Acid Yellow 1, Yellow 10, Acid Yellow 23, Acid Yellow 36, Acid Orange 7, Acid Red 33, Acid Red 52, Pigment Red 57:1, Acid Blue 7, Acid Green 50, Acid Violet 43, Acid Black 1, Acid Black 52, Bromphenolblau und Tetrabromphenolblau bekannten Verbindungen.

Bevorzugte kationische direktziehende Farbstoffe sind kationische Triphenylmethanfarbstoffe, wie beispielsweise Basic Blue 7, Basic Blue 26, Basic Violet 2 und Basic Violet 14, aromatische Systeme, die mit einer quaternären Stickstoffgruppe substituiert sind, wie beispielsweise Basic Yellow 57, Basic Red 76, Basic Blue 99, Basic Brown 16 und Basic Brown 17, sowie direktziehende Farbstoffe, die einen Heterocyclus enthalten, der mindestens ein quaternäres Stickstoffatom aufweist, insbesondere Basic Yellow 87, Basic Orange 31 und Basic Red 51. Die kationischen direktziehenden Farbstoffe, die unter dem Warenzeichen Arianor vertrieben werden, sind erfindungsgemäß ebenfalls bevorzugte kationische direktziehende Farbstoffe.

Als nichtionische direktziehende Farbstoffe eignen sich insbesondere nichtionische Nitro- und Chinonfarbstoffe und neutrale Azofarbstoffe. Bevorzugte nichtionische direktziehende Farbstoffe sind die unter den internationalen Bezeichnungen bzw. Handelsnamen HC Yellow 2, HC Yellow 4, HC Yellow 5, HC Yellow 6, HC Yellow 12, HC Orange 1, Disperse Orange 3, HC Red 1, HC Red 3, HC Red 10, HC Red 11, HC Red 13, HC Red BN, HC Blue 2, HC Blue 11, HC Blue 12, Disperse Blue 3, HC Violet 1, Disperse Violet 1, Disperse Violet 4, Disperse Black 9 bekannten Verbindungen, sowie 1,4-Diamino-2-nitrobenzol, 2-Amino-4-nitrophenol, 1,4-Bis-(2-hydroxyethyl)-amino-2-nitrobenzol, 3-Nitro-4-(2-hydroxyethyl)aminophenol, 2-(2-Hydroxyethyl)amino-4,6-dinitrophenol, 4-[(2-Hydroxyethyl)amino]-3-nitro-1-methylbenzol, 1-Amino-4-(2-hydroxyethyl)amino-5-chlor-2-nitrobenzol, 4-Amino-3-nitrophenol, 1-(2'-Ureidoethyl)amino-4-nitrobenzol, 2-[(4-Amino-2-nitrophenyl)amino]-benzoesäure, 6-Nitro-1,2,3,4-tetrahydrochinoxalin, 2-Hydroxy-1,4-naphthochinon, Pikraminsäure und deren Salze, 2-Amino-6-chloro-4-nitrophenol, 4-Ethylamino-3-nitrobenzoesäure und 2-Chlor-6-ethylamino-4-nitrophenol.

Die Verwendung von Nachbehandlungsmitteln, enthaltend Acetylpyridiniumderivate der Formel (I), ist ebenfalls zur Farbfixierung von Färbungen geeignet, welche mit Färbemitteln erzeugt wurden, die als farbgebende Verbindungen mindestens ein naturanaloges Farbstoffvorprodukt und/oder mindestens ein Oxofarbstoffvorprodukt enthalten.

Als Farbstoffvorstufen naturanaloger Farbstoffe werden bevorzugt solche Indole und Indoline eingesetzt, die mindestens zwei Gruppen ausgewählt aus Hydroxy- und/oder oder Aminogruppen, bevorzugt als Substituent am Sechsring, aufweisen. Diese Gruppen können weitere Substituenten tragen, z. B. in Form einer Veretherung oder Veresterung der Hydroxygruppe oder eine Alkylierung der Aminogruppe. In einer weiteren Ausführungsform enthalten die Färbemittel mindestens ein Indol- und/oder Indolinderivat. Erfindungsgemäße Zusammensetzungen, die Vorstufen naturanaloger Farbstoffe enthalten, werden bevorzugt als luftoxidative Färbemittel verwendet. In dieser Ausführungsform werden die besagten Zusammensetzungen folglich nicht mit einem zusätzlichen Oxidationsmittel versetzt. Die Farbstoffvorstufen naturanaloger Farbstoffe werden jeweils bevorzugt in einer Menge von 0,001 bis 5 Gew.-%, bezogen auf die gesamte Anwendungszubereitung, eingesetzt. Die Gesamtmenge an direktziehenden Farbstoffen beträgt vorzugsweise höchstens 3 Gew.-%.

Bevorzugte Derivate des Indolins sind 5,6-Dihydroxyindolin, N-Methyl-5,6-dihydroxyindolin, N-Ethyl-5,6-dihydroxyindolin, N-Propyl-5,6-dihydroxyindolin, N-Butyl-5,6-dihydroxyindolin sowie 5,6-Dihydroxyindolin-2-carbonsäure und besonders bevorzugt 5,6-Dihydroxyindolin. Bevorzugte Derivate des Indols sind 5,6-Dihydroxyindol, N-Methyl-5,6-dihydroxyindol, N-Ethyl-5,6-dihydroxyindol, N-Propyl-5,6-dihydroxyindol, N-Butyl-5,6-dihydroxyindol, 5,6-Dihydroxyindol-2-carbonsäure sowie insbesondere 5,6-Dihydroxyindol.

Eine weitere Möglichkeit zur Farbgebung bietet die Verwendung von Färbemitteln, welche sogenannte Oxofarbstoffvorprodukte enthalten. Als erfindungsgemäße farbverändernde Komponente können daher in einer weiteren Ausführungsform auch Oxofarbstoffvorprodukte eingesetzt werden. Oxofarbstoffvorprodukte werden bevorzugt als Kombination aus mindestens einer Verbindung (Oxo1), die mindestens eine reaktive Carbonylgruppe enthält, mit mindestens einer Verbindung (Oxo2), ausgewählt aus C,H-aciden Verbindungen und/oder aus Verbindungen mit primärer oder sekundärer Aminogruppe oder Hydroxygruppe, eingesetzt.

Die vorgenannten Komponenten (Oxo1) und (Oxo2) sind im Allgemeinen selbst keine Farbstoffe, und eignen sich daher jede für sich genommen allein nicht zur Färbung keratinhaltiger Fasern. In Kombination bilden sie in dem nichtoxidativen Prozess der sogenannten Oxofärbung die eigentlichen Farbstoffe aus. Die resultierenden Färbungen besitzen teilweise Farbechtheiten auf der keratinhaltigen Faser, die mit denen der Oxidationsfärbung vergleichbar sind.

Es ist jedoch erfindungsgemäß bevorzugt, wenn (Oxo2) nur unter C,H-aciden Verbindungen ausgewählt werden. Die voranstehend genannten Verbindungen (Oxo1) sowie (Oxo2) werden, wenn sie zum Einsatz kommen, jeweils vorzugsweise in einer Menge von 0,001 bis 10 Gew.-%, insbesondere von 0,01 bis 5 Gew.-%, jeweils bezogen auf Gesamtgewicht des anwendungsbereiten Mittels, verwendet.

Es ist nicht erforderlich, dass Oxidationsfarbstoffvorprodukte, direktziehende Farbstoffe, Oxofarbstoffvorprodukte oder naturanaloge Farbstoffe jeweils einheitliche Verbindungen darstellen. Vielmehr können, bedingt durch die Herstellungsverfahren für die einzelnen Farbstoffe, in untergeordneten Mengen noch weitere Komponenten enthalten

sein, soweit diese nicht das Färbeergebnis nachteilig beeinflussen oder aus anderen Gründen, z. B. toxikologischen, ausgeschlossen werden müssen.

Die erfindungsgemäße Verwendung des Nachbehandlungsmittels zur Farbfixierung erfolgt im Anschluss an die, bevorzugt oxidative, Färbung der Haare.

Ein weiterer Gegenstand der vorliegenden Erfindung ist daher ein Verfahren zur Färbung und zur Nachbehandlung von menschlichen Haaren, welches dadurch gekennzeichnet ist, dass

a) in einem ersten Schritt die Haare einer Färbung mit einen farbgebenden Mittel unterworfen werden,
b) in einem zweiten Schritt die Farbträgermasse aus den Haaren ausgewaschen und die Haare anschließend fakultativ getrocknet werden und
c) anschließend in einem dritten Schritt die Haare einer Nachbehandlung mit einem kosmetischen Haarbehandlungsmittel unterworfen werden, welches in einem kosmetischen Träger mindestens ein Acetylpyridiniumderivat der Formel (I),

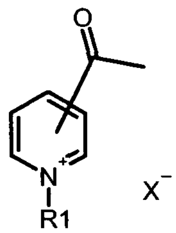

(I),

worin

R1 für eine $C_1$-$C_6$-Alkylgruppe, eine $C_2$-$C_6$-Alkenylgruppe, eine $C_2$-$C_6$-Hydroxyalkylgruppe, eine $C_1$-$C_6$-Alkoxy-$C_2$-$C_6$-alkylgruppe, eine Carboxy-$C_2$-$C_6$-alkylgruppe, eine Aryl-$C_1$-$C_6$-alkylgruppe, eine Heteroaryl-$C_1$-$C_6$-alkylgruppe, eine Arylgruppe oder eine Heteroarylgruppe und
$X^-$ für ein physiologisch verträgliches Anion stehen,

enthält.

Eine besondere Ausführungsform dieses Verfahrens ist dadurch gekennzeichnet ist, dass in einem ersten Schritt a) die Haare einer oxidativen Färbung mit einem oxidativen Färbemittel als farbgebendes Mittel unterworfen werden. Der erste Schritt a) ist dann durch eine oxidative Färbung von menschlichen Haaren gekennzeichnet. Dazu wird als oxidatives Färbemittel eine anwendungsbereite Färbezubereitung, enthaltend in einem kosmetischen Träger mindestens ein Oxidationsfarbstoffvorprodukt sowie gegebenenfalls ein Oxidationsmittel, auf die Haare aufgetragen und für eine Einwirkzeit von 5 bis 45 min auf dem Haar belassen.

Das oxidative Färbemittel kann dabei unmittelbar vor der Anwendung durch Vermischen einer Zubereitung, enthaltend in einem kosmetischen Träger mindestens ein Oxidationsfarbstoffvorprodukt, mit einer Oxidationsmittelzubereitung hergestellt werden.

Bevorzugt beträgt die Einwirkzeit der anwendungsbereiten Färbezubereitung 5 bis 45 min, insbesondere 10 bis 40 min, besonders bevorzugt 15 bis 35 min.

Während der Einwirkzeit des Mittels auf der Faser kann es vorteilhaft sein, den Färbevorgang durch Wärmezufuhr zu unterstützen. Die Wärmezufuhr kann durch eine externe Wärmequelle, wie z.B. warme Luft eines Warmluftgebläses, als auch, insbesondere bei einer Haarfärbung am lebenden Probanden, durch die Körpertemperatur des Probanden erfolgen. Bei letzterer Möglichkeit wird üblicherweise die zu färbende Partie mit einer Haube abgedeckt. Eine Einwirkphase bei Raumtemperatur ist ebenfalls erfindungsgemäß. Insbesondere liegt die Temperatur während der Einwirkzeit zwischen 20 °C und 40 °C, insbesondere zwischen 25 °C und 38 °C. Die oxidativen Färbemittel ergeben bereits bei physiologisch verträglichen Temperaturen von unter 45°C intensive Färbungen.

Nach Ende der Einwirkzeit wird in einem zweiten Schritt b) die verbleibende Farbträgermasse mit Wasser oder einem Reinigungsmittel aus dem Haar gespült. Als Reinigungsmittel kann dabei insbesondere handelsübliches Shampoo dienen, wobei insbesondere dann auf das Reinigungsmittel verzichtet werden kann und der Ausspülvorgang mit Leitungswasser erfolgen kann, wenn das Färbemittel einen stark tensidhaltigen Träger besitzt.

Die Haare können vor dem dritten Verfahrensschritt fakultativ getrocknet werden. Die Nachbehandlung kann jedoch auch auf feuchtem Haar erfolgen.

Das erfindungsgemäße Verfahren ist dadurch gekennzeichnet, dass zwischen dem Ende des zweiten Verfahrensschritt und der Nachbehandlung des dritten Verfahrensschritts nicht zuviel Zeit verstreicht. Üblicherweise schließt sich Nachbehandlung weniger als 60 min, bevorzugt weniger als 30 min, insbesondere weniger als 10 min und besonders

bevorzugt unmittelbar anschließend an den zweiten Verfahrensschritt an.

Im dritten Verfahrensschritt c) wird das erfindungsgemäße Nachbehandlungsmittel auf die noch nassen oder inzwischen getrockneten Haare aufgetragen und dort für 5 bis 45 min, bevorzugt 5 bis 30 min, belassen. Während der Einwirkzeit des Nachbehandlungsmittels auf der Faser kann es ebenfalls vorteilhaft sein, den Behandlungsvorgang durch Wärmezufuhr zu unterstützen. Insbesondere liegt die Temperatur während der Einwirkzeit zwischen 20 °C und 40 °C, insbesondere zwischen 25 °C und 38 °C.

Wird das Nachbehandlungsmittel im Sinne eines "leave on" Produktes angewendet, erfolgt nach der Nachbehandlung kein weiterer Spülvorgang. In einer weiteren Ausführungsform kann das Nachbehandlungsmittel aber auch in Form eines "rinse off" Produktes Anwendung finden. In diesem Fall schließt sich ein weiterer Auswaschvorgang als zusätzlicher Verfahrensschritt an. Hierbei gelten die Ausführungen des zweiten Verfahrensschritts b) analog.

Die, bevorzugt oxidativen, Färbemittel und die Nachbehandlungsmittel der vorgenannten Erfindungsgegenstände können weiterhin zusätzliche Wirk-, Hilfs- und Zusatzstoffe enthalten.

Vorzugsweise werden die Mittel als fließfähigen Zubereitung bereitgestellt und ihm daher zusätzlich ein Emulgator bzw. ein Tensid zugesetzt, wobei oberflächenaktive Substanzen je nach Anwendungsgebiet als Tenside oder als Emulgatoren bezeichnet werden und aus anionischen, kationischen, zwitterionischen, amphoteren und nichtionischen Tensiden und Emulgatoren ausgewählt sind.

Erfindungsgemäß bevorzugte Mittel sind dadurch gekennzeichnet, dass das Mittel zusätzlich mindestens ein anionisches Tensid enthält. Bevorzugte anionische Tenside sind Fettsäuren, Alkylsulfate, Alkylethersulfate und Ethercarbonsäuren mit 10 bis 20 C-Atomen in der Alkylgruppe und bis zu 16 Glykolethergruppen im Molekül. Die anionischen Tenside werden in Mengen von 0,1 bis 45 Gew.%, bevorzugt 1 bis 30 Gew.% und ganz besonders bevorzugt von 1 bis 15 Gew.%, bezogen auf die Gesamtmenge des anwendungsbereiten Mittels, eingesetzt.

Erfindungsgemäß bevorzugte Mittel sind dadurch gekennzeichnet, dass das Mittel zusätzlich mindestens ein zwitterionisches Tensid enthält. Besonders geeignete zwitterionische Tenside sind die sogenannten Betaine und N-Alkyl-N,N-dimethylammonium-glycinate, N-Acyl-aminopropyl-N,N-dimethylammoniumglycinate, und 2-Alkyl-3-carboxymethyl-3-hydroxyethyl-imidazoline. Ein bevorzugtes zwitterionisches Tensid ist das unter der INCI-Bezeichnung Cocamidopropyl Betaine bekannte Fettsäureamid-Derivat.

Erfindungsgemäß bevorzugte Mittel sind dadurch gekennzeichnet, dass das Mittel zusätzlich mindestens ein amphoteres Tensid enthält. Beispiele für geeignete amphotere Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren. Besonders bevorzugte amphotere Tenside sind das N-Kokosalkylaminopropionat, das Kokosacylaminoethylaminopropionat und das $C_{12}$-$C_{18}$-Acylsarcosin.

Weiterhin hat es sich als vorteilhaft erwiesen, wenn die Mittel weitere, nichtionogene grenzflächenaktive Stoffe, enthalten. Als bevorzugte nichtionische Tenside haben sich Alkylpolyglykoside sowie Alkylenoxid-Anlagerungsprodukte an gesättigte lineare Fettalkohole und Fettsäuren mit jeweils 2 bis 30 Mol Ethylenoxid pro Mol Fettalkohol bzw. Fettsäure erwiesen. Zubereitungen mit hervorragenden Eigenschaften werden ebenfalls erhalten, wenn sie als nichtionische Tenside Fettsäureester von ethoxyliertem Glycerin enthalten.

Die nichtionischen, zwitterionischen oder amphoteren Tenside werden in Mengen von 0,1 bis 45 Gew.%, bevorzugt 1 bis 30 Gew.% und ganz besonders bevorzugt von 1 bis 15 Gew.%, bezogen auf die Gesamtmenge der anwendungsbereiten Mittel, eingesetzt.

Erfindungsgemäß geeignete Mittel können auch kationische Tenside vom Typ der quartären Ammoniumverbindungen, der Esterquats und der Amidoamine enthalten. Bevorzugte quaternäre Ammoniumverbindungen sind Ammoniumhalogenide sowie die unter den INCI-Bezeichnungen Quaternium-27 und Quaternium-83 bekannten Imidazolium-Verbindungen. Weitere erfindungsgemäß verwendbare kationische Tenside stellen die quaternisierten Proteinhydrolysate dar. Eine erfindungsgemäß besonders geeignete Verbindung aus der Amidoamine stellt das unter der Bezeichnung Tegoamid® S 18 im Handel erhältliche Stearamidopropyl-dimethylamin dar. Bevorzugte Esterquats sind quaternierte Estersalze von Fettsäuren mit Triethanolamin, quaternierte Estersalze von Fettsäuren mit Diethanolalkylaminen und quaternierten Estersalzen von Fettsäuren mit 1,2-Dihydroxypropyldialkylaminen. Solche Produkte werden beispielsweise unter den Warenzeichen Stepantex®, Dehyquart® und Armocare® vertrieben. Die kationischen Tenside sind in den erfindungsgemäß verwendeten Mitteln bevorzugt in Mengen von 0,05 bis 10 Gew.-%, bezogen auf das gesamte Mittel, enthalten. Mengen von 0,1 bis 5 Gew.-% sind besonders bevorzugt.

Ferner können die erfindungsgemäßen Mittel weitere Wirk-, Hilfs- und Zusatzstoffe enthalten, wie beispielsweise nichtionische Polymere wie beispielsweise Vinylpyrrolidinon/Vinylacrylat-Copolymere, Polyvinylpyrrolidinon, Vinylpyrrolidinon/Vinylacetat-Copolymere, Polyethylenglykole und Polysiloxane; zusätzliche Silikone wie flüchtige oder nicht flüchtige, geradkettige, verzweigte oder cyclische, vernetzte oder nicht vernetzte Polyalkylsiloxane (wie Dimethicone oder Cyclomethicone), Polyarylsiloxane und/oder Polyalkylarylsiloxane, insbesondere Polysiloxane mit organofunktionelle Gruppen, wie substituierten oder unsubstituierten Aminen (Amodimethicone), Carboxyl-, Alkoxy- und/oder Hydroxylgruppen (Dimethiconcopolyole), lineare Polysiloxan(A)-Polyoxyalkylen(B)-Blockcopolymere, gepfropften Silikonpolymere; kationische Polymere wie quaternisierte Celluloseether, Polysiloxane mit quaternären Gruppen, Dimethyldiallylammoniumchlorid-Polymere, Acrylamid-Dimethyldiallyl-ammoniumchlorid-Copolymere, mit Diethylsulfat quaternierte Dimethylamino-ethylmethacrylat-Vinylpyrrolidinon-Copolymere, Vinylpyrrolidinon-Imidazoliniummethochlorid-Copolymere und quaternierter Polyvinylalkohol; zwitterionische und amphotere Polymere; anionische Polymere wie beispielsweise Polyacrylsäuren oder vernetzte Polyacrylsäuren; Strukturanten wie Glucose, Maleinsäure und Milchsäure, haarkonditionierende Verbindungen wie Phospholipide, beispielsweise Lecitin und Kephaline; Parfümöle, Dimethylisosorbid und Cyclodextrine; faserstrukturverbessernde Wirkstoffe, insbesondere Mono-, Di- und Oligosaccharide wie beispielsweise Glucose, Galactose, Fructose, Fruchtzucker und Lactose; Farbstoffe zum Anfärben des Mittels; Antischuppenwirkstoffe wie Piroctone Olamine, Zink Omadine und Climbazol; Aminosäuren und Oligopeptide , insbesondere Arginin und/oder Serin; Proteinhydrolysate auf tierischer und/oder pflanzlicher Basis, wie beispielsweise Elastin-, Kollagen-, Keratin-, Seiden- und Milcheiweiß-Proteinhydrolysate, oder Mandel-, Reis-, Erbsen-, Kartoffel-und Weizenproteinhydrolysate, sowie in Form ihrer Fettsäure-Kondensationsprodukte oder gegebenenfalls anionisch oder kationisch modifizierten Derivate; pflanzliche Öle, wie Macadamianussöl, Palmöl, Amaranthsamenöl, Pfirsichkernöl, Avocadoöl, Olivenöl, Kokosöl, Rapsöl, Sesamöl, Jojobaöl, Sojaöl, Erdnussöl, Nachtkerzenöl und Teebaumöl; Lichtschutzmittel, wie derivatisierte Benzophenone, Zimtsäure-Derivate und Triazine; Wirkstoffe wie Panthenol, Pantothensäure, Pantolacton, Allantoin, Pyrrolidinoncarbonsäuren und deren Salze sowie Bisabolol; Polyphenole, insbesondere Hydroxyzimtsäuren, 6,7-Dihydroxycumarine, Hydroxybenzoesäuren, Catechine, Tannine, Leukoanthocyanidine, Anthocyanidine, Flavanone, Flavone und Flavonole; Ceramide oder Pseudoceramide; Vitamine, Provitamine und Vitaminvorstufen, insbesondere der Gruppen A, $B_3$, $B_5$, $B_6$, C, E, F und H; Pflanzenextrakte wie beispielsweise die Extrakte aus Aloe Vera, Angelica, Anis, Aprikose, Benzoe, Bergamotte, Birke, Brennnessel, Calmus, Cassis, Costus, Eibisch, Eichenrinde, Elemi, Estragon, Fichtennadel, Galbanum, Geranium, Ginseng, Grapefruit, Guajakholz , grünem Tee, Hamamelis, Hauhechel, Hopfen, Huflattich, Ingwerwurzel, Iris, Jasmin, Kamille, Kardamon, Klee, Klettenwurzel, Kiefer, Kiwi, Kokosnuss, Koriander, Kümmel, Latschen, Lavendel, Lemongras, Lilie, Limone, Lindenblüten, Litchi, Macis, Malve, Mandel, Mango, Melisse, Melone, Meristem, Myrrhe, Neroli, Olibanum, Opoponax, Orange, Patchouli, Petitgrain, Pinie, Quendel, Rooibos, Rosen, Rosmarin, Rosskastanie, Sandelholz, Salbei, Schachtelhalm, Schafgarbe, Sellerie, Tanne, Thymian, Wacholder, Weinblättern, Weißdorn, Weizen, Wiesenschaumkraut, Ylang-Ylang, Zeder und Zitrone; Fette und Wachse wie Fettalkohole, Bienenwachs, Montanwachs und Paraffine; Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate; Trübungsmittel wie Latex, Styrol/PVP- und Styrol/Acrylamid-Copolymere; Periglanzmittel wie Ethylenglykolmono- und -distearat sowie PEG-3-distearat; Pigmente sowie Treibmittel wie Propan-Butan-Gemische, $N_2O$, Dimethylether, $CO_2$ und Luft

Die Auswahl dieser weiteren Stoffe wird der Fachmann gemäß der gewünschten Eigenschaften der Mittel treffen. Bezüglich weiterer fakultativer Komponenten sowie der eingesetzten Mengen dieser Komponenten wird ausdrücklich auf die dem Fachmann bekannten einschlägigen Handbücher, z. B. Kh. Schrader, Grundlagen und Rezepturen der Kosmetika, 2. Auflage, Hüthig Buch Verlag, Heidelberg, 1989, verwiesen. Die zusätzlichen Wirk- und Hilfsstoffe werden in den erfindungsgemäßen Mitteln bevorzugt in Mengen von jeweils 0,0001 bis 10 Gew.-%, insbesondere von 0,0005 bis 5 Gew.-%, bezogen auf das Gesamtgewicht der Anwendungsmischung, eingesetzt.

Eine erfindungsgemäß bevorzugte Ausführungsform der vorliegenden Erfindung besteht darin, dass das anwendungsbereite oxidative Färbemittel des ersten Verfahrensschritts a) einen pH-Wert zwischen 6 und 12, insbesondere zwischen 7 und 11,5, insbesondere bevorzugt zwischen 8 und 11, besitzt.

Gemäß einer weiteren erfindungsgemäß bevorzugten Ausführungsform der vorliegenden Erfindung besitzt das Nachbehandlungsmittel, enthaltend mindestens ein Acetylpyridiniumderivat der Formel (I), des dritten Verfahrensschritts c) einen pH-Wert zwischen 2,0 und 8,0, bevorzugt zwischen 2,5 und 7,5 und insbesondere bevorzugt zwischen 3,0 und 7,0. Bei den pH-Werten im Sinne der vorliegenden Erfindung handelt es sich um pH-Werte, die bei einer Temperatur von 22 °C gemessen wurden.

Üblicherweise wird der pH-Wert mit pH-Stellmitteln eingestellt. Zur Einstellung des pH-Werts sind dem Fachmann in der Kosmetik gängige Acidifizierungs- und Alkalisierungsmittel geläufig. Die zur Einstellung des pH-Wertes verwendbaren Alkalisierungsmittel werden typischerweise gewählt aus anorganischen Salzen, insbesondere der Alkali- und Erdalkalimetalle, organischen Alkalisierungsmitteln, insbesondere Aminen, basische Aminosäuren und Alkanolaminen, und Ammoniak. Erfindungsgemäß bevorzugte Acidifizierungsmittel sind Genuss-Säuren, wie beispielsweise Zitronensäure, Essigsäure, Äpfelsäure oder Weinsäure, sowie verdünnte Mineralsäuren.

Erfindungsgemäß einsetzbare, organische Alkalisierungsmittel werden bevorzugt aus der Gruppe ausgewählt, die gebildet wird, aus 2-Aminoethan-1-ol (Monoethanolamin), 3-Aminopropan-1-ol, 4-Aminobutan-1-ol, 5-Aminopentan-1-ol, 1-Aminopropan-2-ol (Monoisopropanolamin), 1-Aminobutan-2-ol, 1-Aminopentan-2-ol, 1-Aminopentan-3-ol, 1-Aminopentan-4-ol, 2-Amino-2-methyl-propanol, 2-Amino-2-methylbutanol, 3-Amino-2-methylpropan-1-ol, 1-Amino-2-methylpropan-2-ol, 3-Aminopropan-1,2-diol, 2-Amino-2-methylpropan-1,3-diol, 2-Amino-2-ethyl-1,3-propandiol, N,N-Dimethyl-ethanolamin, Methylglucamin, Triethanolamin, Diethanolamin und Triisopropanolamin. Erfindungsgemäß bevorzugte Alkanolamine sind Monoethanolamin und Triethanolamin. Erfindungsgemäße, anorganische Alkalisierungsmittel werden bevorzugt ausgewählt aus der Gruppe, die gebildet wird aus Natriumhydroxid, Kaliumhydroxid, Calciumhydroxid, Bariumhydroxid, Natriumphosphat, Kaliumphosphat, Natriumsilicat, Kaliumsilicat, Natriumcarbonat und Kaliumcarbonat.

Ganz besonders bevorzugt sind Natriumhydroxid und/oder Kaliumhydroxid. Die als erfindungsgemäßes Alkalisierungsmittel einsetzbaren basischen Aminosäuren werden bevorzugt ausgewählt aus der Gruppe, die gebildet wird aus L-Arginin, D-Arginin, D/L-Arginin, L-Lysin, D-Lysin, D/L-Lysin, besonders bevorzugt L-Arginin, D-Arginin, D/L-Arginin als ein Alkalisierungsmittel im Sinne der Erfindung eingesetzt. Schließlich ist ein weiteres bevorzugtes Alkalisierungsmittel Ammoniak.

Die Ausführungsformen des ersten Erfindungsgegenstands gelten mutatis mutandis für das Verfahren des zweiten Erfindungsgegenstands.

Die erfindungsgemäße Verwendung der Nachbehandlungsmittel zur Verbesserung des Farberhalts gegebenenfalls oxidativer Färbungen findet bevorzugt zeitnah nach dem Färbevorgang statt. Es ist daher aus Gründen der Anwendungserleichterung und Verpackungsökonomie zweckmäßig, dem Anwender beide Mittel gemeinsam zur Verfügung zu stellen.

Ein weiterer Gegenstand der vorliegenden Erfindung ist daher eine Mehrkomponentenverpackungseinheit (Kit-of-Parts), umfassend mindestens zwei getrennt voneinander konfektionierte Komponenten, welche dadurch gekennzeichnet ist, dass

i) die erste Komponente eine Färbezubereitung (A) ist, enthaltend in einem kosmetischen Träger mindestens ein farbgebendes Mittel, und dass
ii) die zweite Komponente ein Nachbehandlungsmittel (C) ist, welches in einem kosmetischen Träger mindestens ein Acetylpyridiniumderivat der Formel (I),

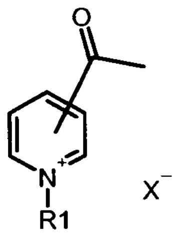

(I),

worin

R1 für eine $C_1$-$C_6$-Alkylgruppe, eine $C_2$-$C_6$-Alkenylgruppe, eine $C_2$-$C_6$-Hydroxyalkylgruppe, eine $C_1$-$C_6$-Alkoxy-$C_2$-$C_6$-alkylgruppe, eine Carboxy-$C_2$-$C_6$-alkylgruppe, eine Aryl-$C_1$-$C_6$-alkylgruppe, eine Heteroaryl-$C_1$-$C_6$-alkylgruppe, eine Arylgruppe oder eine Heteroarylgruppe und
$X^-$ für ein physiologisch verträgliches Anion stehen,

enthält.

Bevorzugt enthält die Färbezubereitung (A) als farbgebendes Mittel mindestens ein Oxidationsfarbstoffvorprodukt und/oder mindestens einen direktziehenden Farbstoff. Besonders bevorzugt handelt es sich bei Färbezubereitung (A) um ein Oxidationsfärbemittel.

Eine Ausführungsform dieses Erfindungsgegenstands ist daher eine Mehrkomponentenverpackungseinheit (Kit-of-Parts), umfassend mindestens drei getrennt voneinander konfektionierte Komponenten, welche dadurch gekennzeichnet ist, dass

i) die erste Komponente eine Färbezubereitung (A) ist, enthaltend in einem kosmetischen Träger mindestens ein Oxidationsfarbstoffvorprodukt,
ii) die zweite Komponente eine Oxidationsmittelzubereitung (B) ist, enthaltend in einem kosmetischen Träger mindestens ein Oxidationsmittel, und
iii) die dritte Komponente ein Nachbehandlungsmittel (C) ist, welches in einem kosmetischen Träger mindestens ein Acetylpyridiniumderivat der Formel (I),

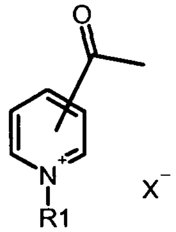

(I),

worin

R1 für eine $C_1$-$C_6$-Alkylgruppe, eine $C_2$-$C_6$-Alkenylgruppe, eine $C_2$-$C_6$-Hydroxyalkylgruppe, eine $C_1$-$C_6$-Alkoxy-$C_2$-$C_6$-alkylgruppe, eine Carboxy-$C_2$-$C_6$-alkylgruppe, eine Aryl-$C_1$-$C_6$-alkylgruppe, eine Heteroaryl-$C_1$-$C_6$-alkylgruppe, eine Arylgruppe oder eine Heteroarylgruppe und

$X^-$ für ein physiologisch verträgliches Anion stehen,

enthält.

Die Komponenten der Mehrverpackungseinheit werden getrennt voneinander in räumlich unterschiedlichen Behältern oder Containern konfektioniert.

Der Begriff Behältern" kennzeichnet hierbei eine Aufnahmemöglichkeit, unabhängig von deren Form, Material oder Verschluss, welche die Möglichkeit beinhaltet, Stoffe oder Stoffgemische zu beinhalten. Der Begriff Behälter umfasst daher, ohne darauf beschränkt zu sein, das Innere einer Tube, eines Beutels oder Sackes, eines Kanisters, einer Dose, einer Wanne, einer Flasche, eines Glases oder eines Paketes, eines Kartons, einer Box, eines Umschlages oder eines anderen Behältnisses. Die Behälter können mit einer wiederverschließbaren Öffnung wie einem Schraubverschluss versehen sein. Dies kann insbesondere dann von Vorteil sein, wenn mehrere Mittel vor der Anwendung durch Schütteln innig miteinander vermischt werden sollten.

Die Komponenten der oxidativen Färbezubereitung können in einem Doppelkammer-Behälter mit getrennter oder gemeinsamer Öffnung enthalten sein. Es ist jedoch bevorzugt, sie auf verschiedene Behälter aufzuteilen und den Verbraucher anzuleiten, sie vor der Anwendung miteinander zu mischen.

Bevorzugt enthält die Mehrkomponentenverpackungseinheit (Kit-of-Parts) zusätzlich eine Gebrauchsanleitung. Die Gebrauchsanleitung enthält insbesondere Informationen, Erläuterungen und gegebenenfalls Illustrationen für den Verbraucher (m/f) zur Anwendung der Mittel aus den Behältern der Verpackungseinheit in einem Verfahren gemäß dem zweiten Erfindungsgegenstand. Darüber hinaus kann es bevorzugt sein, wenn weiterhin eine Anmischhilfe, wie beispielsweise eine Schale, eine Applikationshilfe, wie beispielsweise ein Kamm oder ein Pinsel, und/oder eine persönliche Schutzausrüstung, wie beispielsweise Einweg-Handschuhe dem Kit beigefügt sind.

Bezüglich bevorzugter Ausführungsformen der erfindungsgemäßen Mehrkomponentenverpackungseinheit gelten mutatis mutandis die Ausführungsformen der vorangegangenen Erfindungsgegenstände.

Die folgenden Beispiele erläutern die Erfindung, ohne sie jedoch darauf einzuschränken.

Beispiele:

1. Herstellung der Färbecreme

Es wurde die folgenden Färbecreme hergestellt:

| | | |
|---|---|---|
| Hydrenol D | [1] | 8,5 g |
| Lorol techn. | [2] | 2,0 g |
| Texapon NSO-UP | [3] | 20,0 g |
| Dehyton K | [4] | 12,5 g |
| Eumulgin B2 | [5] | 0,75 g |
| Natriumsulfit | | 1,0 g |
| Ammoniumsulfat | | 1,0 g |
| 4,5-Diamino-1-(2-hydroxyethyl)pyrazol, Sulfat | | 3 mmol |
| Resorcin | | 3 mmol |
| Propylenglykol | | 5,0 g |

(fortgesetzt)

| | |
|---|---|
| Wasser | ad 100 |

Es wurden die folgenden Handelsprodukte eingesetzt:

[1] INCI-Bezeichnung: Cetearyl alcohol (Cognis)
[2] INCI-Bezeichnung: Coconut alcohol (Cognis)
[3] INCI-Bezeichnung: Sodium Laureth Sulfate; ca. 27,5% Aktivsubstanz (Cognis)
[4] INCI-Bezeichnung: Cocamidopropyl Betaine (Cognis)
[5] INCI-Bezeichnung: Ceteareth-20 (Cognis)

Hydrenol D und Lorol wurden zusammen mit Texapon NSO, Dehyton K und Eumulgin B2 bei 80°C aufgeschmolzen. Dann wurde die Schmelze mit dem in einem Teil des Wassers gelösten Natriumsulfit und Ammoniumsulfat emulgiert. Oxidationsfarbstoffvorprodukte wurden in Propylenglykol und einem weiteren Teil der angegebenen Wassermenge unter Erhitzen gelöst und unter Rühren hinzugegeben. Dann wurde die Formulierung auf 100 % mit Wasser aufgefüllt und kalt gerührt.

2. Vermischen mit der Entwicklerdispersion und Anwendung

Die auf diese Weise erhaltene Färbecreme wurde im Verhältnis 2:1 mit einer Oxidationsmittelzubereitung mit einem Wasserstoffperoxidgehalt von 3 Gew.-% vermischt. Für den Färbeprozess wurden geschädigte Strähnen (zweimal blondiert und zweimal dauergewellt; Kerling, Euronaturhaar weiß) verwendet. Auf jede Strähne wurde jeweils die 4-fache Menge der anwendungsbereiten Mischung appliziert und dort für 30 Minuten bei 32 °C belassen.

3. Auswaschvorgang

Anschließend wurden die Strähnen mit lauwarmem Wasser ausgespült, mit einem handelsüblichen Shampoo nachgewaschen und getrocknet.

4. Nachbehandlung

Für den Nachbehandlungsschritt wurde eine gefärbte Strähne für 15 Minuten bei Raumtemperatur mit der nachfolgenden Lösung behandelt:

| | | |
|---|---|---|
| 4-Acetyl-1-methylpyridinium-p-toluolsuffonat | 1,00 g | |
| Wasser | ad 100 g | (pH-Wert der Lösung: 3,4) |

Eine Referenzsträhne wurde analog mit Wasser behandelt, welches durch Zugabe von Zitronensäure auf einen pH-Wert von 3,4 gebracht wurde.

5. Auswaschvorgang

Anschließend wurden die Strähnen erneut mit lauwarmem Wasser ausgespült und getrocknet.

6. Farbmetrische Vermessung

Die Egalisiersträhnen wurden farbmetrisch mit einem Farbmessgerät der Firma Datacolor, Typ Spectraflash 450 vermessen.

Der L*a*b*-Farbraum wird durch ein dreidimensionales Koordinatensystem beschrieben. Die L*-Achse gibt mit den Endpunkten Schwarz (L = 0) und Weiß (L = 100) die Helligkeit einer Farbe wieder. Die a*-Achse beschreibt den Rot- bzw. Grünanteil einer Farbe, wobei eine Farbe mit hohem Grünanteil einen negativen a-Wert und eine Farbe mit hohem Rotanteil einen positiven a-Wert besitzt. Die b*-Achse beschreibt den Blau- oder Gelbanteil einer Farbe, wobei negative b-Werte für eine Farbe mit hohem Blauanteil und positive b-Werte für eine Farbe mit hohem Gelbanteil stehen.

Die Farbstärke (Chroma) berechnet sich daraus nach der Formel $C = \sqrt{(a^*)^2 + (b^*)^2}$ .

7. Haarwäsche

Zur Simulation des Auswaschvorgangs wurden die Haarsträhnen für 15 Minuten in ein Ultraschallbad der Firma Elma (Typ T 790/H, Stufe 5), welches mit einer wässrigen, 1 Gew.-%-igen Texapon-NSO Lösung befüllt war, eingebracht. Nach dem Trocknen erfolgte eine erneute farbmetrische Vermessung.

8. Ergebnisse

Tabelle 1: Vergleichsbehandlung

| | nach 0 Haarwäschen | | | nach 6 Haarwäschen | | |
|---|---|---|---|---|---|---|
| | a* | b* | $C_0$ | a* | b* | $C_6$ |
| Nachbehandlung mit Wasser | 13,55 | 4,33 | 14,23 | 11,12 | 2,73 | 11,19 |

Tabelle 2: Erfindungsgemäße Nachbehandlung

| | nach 0 Haarwäschen | | | nach 6 Haarwäschen | | |
|---|---|---|---|---|---|---|
| | a* | b* | $C_0$ | a* | b* | $C_6$ |
| Nachbehandlung mit 4-Acetyl-1-methylpyridinium-p-toluolsulfonat-Lsg. | 15,31 | 4,43 | 15,94 | 14,60 | 3,62 | 15,04 |

Der Rotanteil einer Nuance ist umso größer, je höher der entsprechende a*-Wert ist. Bereits direkt nach der Nachbehandlung mit dem erfindungsgemäßen Nachbehandlungsmittel ließ sich im Vergleich zur Referenzsträhne eine stärkere Konservierung der Rotfärbung sowohl visuell beobachten als auch messtechnisch erfassen ($\Delta$a*-Wert = 1,76). Nach 6 Haarwäschen wurde dieser Effekt weiter verstärkt ($\Delta$a*-Wert = 3,48), woraus sich ablesen lässt, dass die Rotanteile der Nuance im Falle der erfindungsgemäßen Nachbehandlung deutlich besser fixiert wurden.

Weiterhin wird aus den Ergebnissen der Tabellen 1 und 2 deutlich, dass der Chromaverlust ($\Delta C = C_0 - C_6$) durch wiederholte Haarwäsche bei der Vergleichsnachbehandlung sehr viel höher ausfällt, als bei dem erfindungsgemäßen Nachbehandlungsmitttel ($\Delta C$ = 3,03, nicht-erfindungsgemäß gegenüber $\Delta C$ = 0,90, erfindungsgemäß).

## Patentansprüche

1. Verwendung eines kosmetischen Nachbehandlungsmittels zur Verbesserung der Waschechtheit von auf dem Haar erzeugten Färbungen und zur Farbfixierung, **dadurch gekennzeichnet, dass** es in einem kosmetischen Träger mindestens ein Acetylpyridiniumderivat der Formel (I),

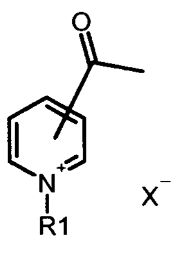

(I),

worin

R1 für eine $C_1$-$C_6$-Alkylgruppe, eine $C_2$-$C_6$-Alkenylgruppe, eine $C_2$-$C_6$-Hydroxyalkylgruppe, eine $C_1$-$C_6$-Alkoxy-$C_2$-$C_6$-alkylgruppe, eine Carboxy-$C_2$-$C_6$-alkylgruppe, eine Aryl-$C_1$-$C_6$-alkylgruppe, eine Heteroaryl-$C_1$-$C_6$-alkylgruppe, eine Arylgruppe oder eine Heteroarylgruppe und
$X^-$ für ein physiologisch verträgliches Anion stehen,

enthält.

2. Verwendung nach Anspruch 1, **dadurch gekennzeichnet, dass** das Nachbehandlungsmittel als Acetylpyridiniumderivat 4-Acetyl-1-methylpyridinium-p-toluolsulfonat und/oder 2-Acetyl-1-methylpyridinium-p-toluolsulfonat enthält.

3. Verwendung nach einem der Ansprüche 1 oder 2 zur Verbesserung der Waschechtheit und zur Farbfixierung von oxidativen Färbungen.

4. Verwendung nach einem der Ansprüche 1 bis 3 zur Verbesserung der Waschechtheit von oxidativen Färbungen, **dadurch gekennzeichnet, dass** diesen Färbungen der Einsatz mindestens eines Oxidationsfarbstoffvorprodukts vom Entwicklertyp, ausgewählt aus der Gruppe, die gebildet wird aus 2,4,5,6-Tetraaminopyrimidin; 4-Hydroxy-2,5,6-triaminopyrimidin, 2-Hydroxy-4,5,6-triaminopyrimidin, 2-Dimethylamino-4,5,6-triaminopyrimidin, 2,4-Dihydroxy-5,6-diaminopyrimidin und 2,5,6-Triaminopyrimidin sowie 4,5-Diamino-1-methylpyrazol, 4,5-Diamino-1-(2-hydroxyethyl)pyrazol, 3,4-Diaminopyrazol, 4,5-Diamino-1-(4'-chlorbenzyl)pyrazol, 4,5-Diamino-1,3-dimethylpyrazol, 4,5-Diamino-3-methyl-1-phenylpyrazol, 4,5-Diamino-1-methyl-3-phenylpyrazol, 4-Amino-1,3-dimethyl-5-hydrazinopyrazol, 1-Benzyl-4,5-diamino-3-methylpyrazol, 4,5-Diamino-3-t-butyl-1-methylpyrazol, 4,5-Diamino-1-t-butyl-3-methylpyrazol, 4,5-Diamino-1-(2-hydroxyethyl)-3-methylpyrazol, 4,5-Diamino-1-ethyl-3-methylpyrazol, 4,5-Diamino-1-ethyl-3-(4-methoxyphenyl)pyrazol, 4,5-Diamino-1-ethyl-3-hydroxymethylpyrazol, 4,5-Diamino-3-hydroxymethyl-1-methylpyrazol, 4,5-Diamino-3-hydroxymethyl-1-isopropylpyrazol, 4,5-Diamino-3-methyl-1-isopropylpyrazol, 4-Amino-5-(2-aminoethyl)amino-1,3-dimethylpyrazol, sowie deren physiologisch verträglichen Salze, zugrunde liegt.

5. Verfahren zur Färbung und Nachbehandlung von menschlichen Haaren, **dadurch gekennzeichnet, dass** die Haare

a) in einem ersten Schritt einer Färbung mit einem farbgebenden Mittel unterworfen werden,
b) die Farbträgermasse in einem zweiten Schritt aus den Haaren ausgewaschen und die Haare anschließend fakultativ getrocknet werden und
c) anschließend die Haare in einem dritten Schritt einer Nachbehandlung mit einem kosmetischen Haarbehandlungsmittel unterworfen werden, welches in einem kosmetischen Träger mindestens ein Acetylpyridiniumderivat der Formel (I),

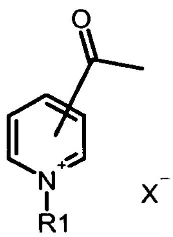

(I),

worin

R1 für eine $C_1$-$C_6$-Alkylgruppe, eine $C_2$-$C_6$-Alkenylgruppe, eine $C_2$-$C_6$-Hydroxyalkylgruppe, eine $C_1$-$C_6$-Alkoxy-$C_2$-$C_6$-alkylgruppe, eine Carboxy-$C_2$-$C_6$-alkylgruppe, eine Aryl-$C_1$-$C_6$-alkylgruppe, eine Heteroaryl-$C_1$-$C_6$-alkylgruppe, eine Arylgruppe oder eine Heteroarylgruppe und
$X^-$ für ein physiologisch verträgliches Anion stehen,

enthält.

6. Verfahren nach Anspruch 5, **dadurch gekennzeichnet, dass** in einem ersten Schritt a) die Haare einer oxidativen Färbung mit einem oxidativen Färbemittel unterworfen werden.

7. Mehrkomponentenverpackungseinheit (Kit-of-Parts), umfassend mindestens zwei getrennt voneinander konfektionierte Komponenten, **dadurch gekennzeichnet, dass**

i) die erste Komponente eine Färbezubereitung (A) ist, enthaltend in einem kosmetischen Träger mindestens ein farbgebendes Mittel, und
ii) die zweite Komponente ein Nachbehandlungsmittel (C) ist, welches in einem kosmetischen Träger mindestens

ein Acetylpyridiniumderivat der Formel (I),

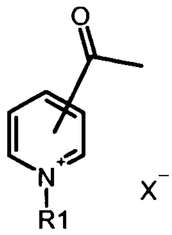

(I),

worin

R1 für eine $C_1$-$C_6$-Alkylgruppe, eine $C_2$-$C_6$-Alkenylgruppe, eine $C_2$-$C_6$-Hydroxyalkylgruppe, eine $C_1$-$C_6$-Alkoxy-$C_2$-$C_6$-alkylgruppe, eine Carboxy-$C_2$-$C_6$-alkylgruppe, eine Aryl-$C_1$-$C_6$-alkylgruppe, eine Heteroaryl-$C_1$-$C_6$-alkylgruppe, eine Arylgruppe oder eine Heteroarylgruppe und
$X^-$ für ein physiologisch verträgliches Anion stehen,

enthält.

**8.** Mehrkomponentenverpackungseinheit (Kit-of-Parts), umfassend mindestens drei getrennt voneinander konfektionierte Komponenten, **dadurch gekennzeichnet, dass**

i) die erste Komponente eine Färbezubereitung (A) ist, enthaltend in einem kosmetischen Träger mindestens ein Oxidationsfarbstoffvorprodukt,
ii) die zweite Komponente eine Oxidationsmittelzubereitung (B) ist, enthaltend in einem kosmetischen Träger mindestens ein Oxidationsmittel, und
iii) die dritte Komponente ein Nachbehandlungsmittel (C) ist, welches in einem kosmetischen Träger mindestens ein Acetylpyridiniumderivat der Formel (I),

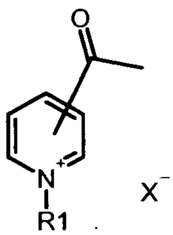

(I),

worin

R1 für eine $C_1$-$C_6$-Alkylgruppe, eine $C_2$-$C_6$-Alkenylgruppe, eine $C_2$-$C_6$-Hydroxyalkylgruppe, eine $C_1$-$C_6$-Alkoxy-$C_2$-$C_6$-alkylgruppe, eine Carboxy-$C_2$-$C_6$-alkylgruppe, eine Aryl-$C_1$-$C_6$-alkylgruppe, eine Heteroaryl-$C_1$-$C_6$-alkylgruppe, eine Arylgruppe oder eine Heteroarylgruppe und
$X^-$ für ein physiologisch verträgliches Anion stehen,

enthält.

## Claims

**1.** Use of a cosmetic post-treatment agent to improve the washing fastness of coloring processes produced on hair and for color fixing, wherein it contains, in a cosmetic carrier, at least one acetylpyridinium derivative of formula (I),

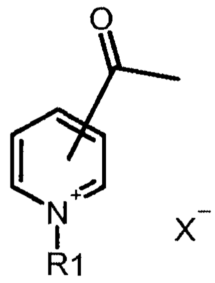

(I),

in which

R1 denotes a $C_1$ to $C_6$ alkyl group, a $C_2$ to $C_6$ alkenyl group, a $C_2$ to $C_6$ hydroxyalkyl group, a $C_1$ to $C_6$ alkoxy-$C_2$ to $C_6$ alkyl group, a carboxy-$C_2$ to $C_6$ alkyl group, an aryl-$C_1$ to $C_6$ alkyl group, a heteroaryl-$C_1$ to $C_6$ alkyl group, an aryl group, or a heteroaryl group, and
$X^-$ denotes a physiologically acceptable anion.

2. The use according to Claim 1, wherein the post-treatment agent contains, as an acetylpyridinium derivative, 4-acetyl-1-methylpyridinium p-toluenesulfonate and/or 2-acetyl-1-methylpyridinium p-toluenesulfonate.

3. The use according to one of Claims 1 or 2 to improve the washing fastness, and for color fixing, of oxidative coloring processes.

4. The use according to one of Claims 1 to 3 to improve the washing fastness of oxidative coloring processes, wherein said coloring processes are based on the use of at least one oxidation dye precursor product of the developer type, selected from the group that is constituted from 2,4,5,6-tetraaminopyrimidine, 4-hydroxy-2,5,6-triaminopyrimidine, 2-hydroxy-4,5,6-triaminopyrimidine, 2-dimethylamino-4,5,6-triaminopyrimidine, 2,4-dihydroxy-5,6-diaminopyrimidine, and 2,5,6-triaminopyrimidine, as well as 4,5-diamino-1-methylpyrazole, 4,5-diamino-1-(2-hydroxyethyl)pyrazole, 3,4-diaminopyrazole, 4,5-diamino-1-(4'-chlorobenzyl)pyrazole, 4,5-diamino-1,3-dimethylpyrazole, 4,5-diamino-3-methyl-1-phenylpyrazole, 4,5-diamino-1-methyl-3-phenylpyrazole, 4-amino-1,3-dimethyl-5-hydrazinopyrazole, 1-benzyl-4,5-diamino-3-methylpyrazole, 4,5-diamino-3-t-butyl-1-methylpyrazole, 4,5-diamino-1-t-butyl-3-methylpyrazole, 4,5-diamino-1-(2-hydroxyethyl)-3-methylpyrazole, 4,5-diamino-1-ethyl-3-methylpyrazole, 4,5-diamino-1-ethyl-3-(4-methoxyphenyl)pyrazole, 4,5-diamino-1-ethyl-3-hydroxymethylpyrazole, 4,5-diamino-3-hydroxymethyl-1-methylpyrazole, 4,5-diamino-3-hydroxymethyl-1-isopropylpyrazole, 4,5-diamino-3-methyl-1-isopropylpyrazole, 4-amino-5-(2-aminoethyl)amino-1,3-dimethylpyrazole, and physiologically acceptable salts thereof.

5. A method for coloring and post-treating human hair, wherein the hair

a) in a first step is subjected to a coloring process using a color-imparting agent,
b) the color-carrying substance is washed out of the hair in a second step, and the hair is then optionally dried, and
c) in a third step, the hair is then subjected to a post-treatment with a cosmetic hair treatment agent that contains, in a cosmetic carrier, at least one acetylpyridinium derivative of formula (I),

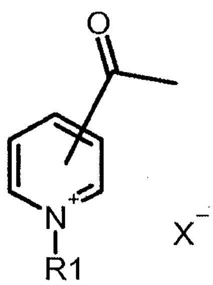

(I),

in which

R1 denotes a $C_1$ to $C_6$ alkyl group, a $C_2$ to $C_6$ alkenyl group, a $C_2$ to $C_6$ hydroxyalkyl group, a $C_1$ to $C_6$

alkoxy-$C_2$ to $C_6$ alkyl group, a carboxy-$C_2$ to $C_6$ alkyl group, an aryl-$C_1$ to $C_6$ alkyl group, a heteroaryl-$C_1$ to $C_6$ alkyl group, an aryl group, or a heteroaryl group, and
$X^-$ denotes a physiologically acceptable anion.

**6.** The method according to Claim 5, wherein in a first step a), the hair is subjected to an oxidative coloring process using an oxidative coloring agent.

**7.** A multi-component packaging unit (kit of parts) encompassing at least two components packaged separately from one another, wherein

i) the first component is a coloring preparation (A) containing, in a cosmetic carrier, at least one color-imparting agent, and
ii) the second component is a post-treatment agent (C) that contains, in a cosmetic carrier, at least one acetylpyridinium derivative of formula (I),

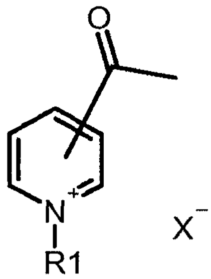
(I),

in which

R1 denotes a $C_1$ to $C_6$ alkyl group, a $C_2$ to $C_6$ alkenyl group, a $C_2$ to $C_6$ hydroxyalkyl group, a $C_1$ to $C_6$ alkoxy-$C_2$ to $C_6$ alkyl group, a carboxy-$C_2$ to $C_6$ alkyl group, an aryl-$C_1$ to $C_6$ alkyl group, a heteroaryl-$C_1$ to $C_6$ alkyl group, an aryl group, or a heteroaryl group, and
$X^-$ denotes a physiologically acceptable anion.

**8.** A multi-component packaging unit (kit of parts) encompassing at least three components packaged separately from one another, wherein

i) the first component is a coloring preparation (A) containing, in a cosmetic carrier, at least one oxidation dye precursor product,
ii) the second component is an oxidizing agent preparation (B) containing, in a cosmetic carrier, at least one oxidizing agent, and
iii) the third component is a post-treatment agent (C) that contains, in a cosmetic carrier, at least one acetylpyridinium derivative of formula (I),

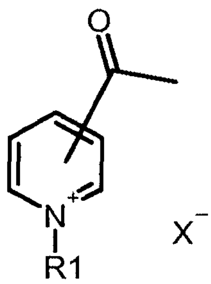
(I),

in which

R1 denotes a $C_1$ to $C_6$ alkyl group, a $C_2$ to $C_6$ alkenyl group, a $C_2$ to $C_6$ hydroxyalkyl group, a $C_1$ to $C_6$ alkoxy-$C_2$ to $C_6$ alkyl group, a carboxy-$C_2$ to $C_6$ alkyl group, an aryl-$C_1$ to $C_6$ alkyl group, a heteroaryl-$C_1$

to $C_6$ alkyl group, an aryl group, or a heteroaryl group, and
$X^-$ denotes a physiologically acceptable anion.

## Revendications

1. Utilisation d'un agent cosmétique de traitement ultérieur pour l'amélioration de la solidité au lavage de teintures générées sur les cheveux et pour la fixation des couleurs, **caractérisée en ce que** l'agent contient, dans un support cosmétique, au moins un dérivé d'acétylpyridinium répondant à la formule (I)

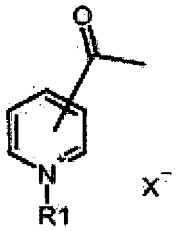

(I),

dans laquelle

R1 représente un groupe alkyle en $C_1$-$C_6$, un groupe alcényle en $C_2$-$C_6$, un groupe hydroxyalkyle en $C_2$-$C_6$, un groupe alcoxy(en $C_1$-$C_6$)alkyle en $C_2$-$C_6$, un groupe carboxyalkyle en $C_2$-$C_6$, un groupe arylalkyle en $C_1$-$C_6$, un groupe hétéroarylalkyle en $C_1$-$C_6$, un groupe aryle ou un groupe hétéroaryle ; et
$X^-$ représente un anion physiologiquement acceptable.

2. Utilisation selon la revendication 1, **caractérisée en ce que** l'agent de traitement ultérieur contient, à titre de dérivé d'acétylpyridinium, le p-toluènesulfonate de 4-acétyl-1-méthylpyridinium et/ou le p-toluènesulfonate de 2-acétyl-1-méthylpyridinium.

3. Utilisation selon la revendication 1 ou 2, pour l'amélioration de la solidité au lavage et pour la fixation des couleurs de teintures oxydatives.

4. Utilisation selon l'une quelconque des revendications 1 à 3, pour l'amélioration de la solidité au lavage de teintures oxydatives **caractérisée en ce que**, à la base de ces teintures, on trouve la mise en oeuvre d'au moins un précurseur de colorant d'oxydation de type développeur choisi parmi le groupe qui est formé par la 2,4,5,6-tétraaminopyrimidine, la 4-hydroxy-2,5,6-triaminopyrimidine, la 2-hydroxy-4,5,6-triaminopyrimidine, la 2-diméthylamino-4,5,6-triaminopyrimidine, la 2,4-dihydroxy-5,6-diaminopyrimidine et la 2,5,6-triaminopyrimidine ainsi que le 4,5-diamino-1-méthylpyrazole, le 4,5-diamino-1-(2-hydroxyéthyl)pyrazole, le 3,4-diaminopyrazole, le 4,5-diamino-1-(4'-chlorobenzyl)pyrazole, le 4,5-diamino-1,3-diméthylpyrazole, le 4,5-diamino-3-méthyl-1-phénylpyrazole, le 4,5-diamino-1-méthyl-3-phénylpyrazole, le 4-amino-1,3-diméthyl-5-hydrazinopyrazole, le 1-benzyl-4,5-diamino-3-méthylpyrazole, le 4,5-diamino-3-t-butyl-1-méthylpyrazole, le 4,5-diamino-1-t-butyl-3-méthylpyrazole, le 4,5-diamino-1-(2-hydroxyéthyl)-3-méthylpyrazole, le 4,5-diamino-1-éthyl-3-méthylpyrazole, le 4,5-diamino-1-éthyl-3-(4-méthoxyphényl)pyrazole, le 4,5-diamino-1-éthyl-3-hydroxyméthylpyrazole, le 4,5-diamino-3-hydroxyméthyl-1-méthylpyrazole, le 4,5-diamino-3-hydroxyméthyl-1-isopropylpyrazole, le 4,5-diamino-3-méthyl-1-isopropylpyrazole, le 4-amino-5-(2-aminoéthyl)amino-1,3-diméthylpyrazole, ainsi que leurs sels physiologiquement acceptables.

5. Procédé pour la teinture et le traitement ultérieur de cheveux humains, **caractérisé en ce qu'**on soumet les cheveux

a) dans une première étape à une teinture avec un agent chromogène ;
b) dans une deuxième étape, on élimine des cheveux par lavage la matière faisant office de chromophore et ensuite, de manière facultative, on sèche les cheveux ; et
c) on soumet ensuite les cheveux, dans une troisième étape, à un traitement ultérieur avec un agent cosmétique pour le traitement capillaire, qui contient, dans un support cosmétique, au moins un dérivé d'acétylpyridinium répondant à la formule (I)

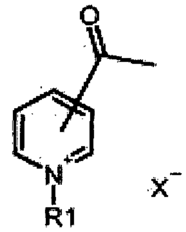

(I),

dans laquelle

R1 représente un groupe alkyle en $C_1$-$C_6$, un groupe alcényle en $C_2$-$C_6$, un groupe hydroxyalkyle en $C_2$-$C_6$, un groupe alcoxy(en $C_1$-$C_6$)alkyle en $C_2$-$C_6$, un groupe carboxyalkyle en $C_2$-$C_6$, un groupe arylalkyle en $C_1$-$C_6$, un groupe hétéroarylalkyle en $C_1$-$C_6$, un groupe aryle ou un groupe hétéroaryle ; et
$X^-$ représente un anion physiologiquement acceptable.

**6.** Procédé selon la revendication 5, **caractérisé en ce qu'**on soumet les cheveux, dans une première étape a), à une teinture par oxydation avec un colorant oxydant.

**7.** Unité de conditionnement à plusieurs composants (kit-of-parts) comprenant au moins deux composants confectionnés séparés l'un de l'autre, **caractérisée en ce que**

i) le premier composant représente une préparation (A) pour la teinture contenant, dans un support cosmétique, au moins un agent chromogène ; et
ii) le deuxième composant représente un agent de traitement ultérieur (C), qui contient, dans un support cosmétique, au moins un dérivé d'acétylpyridinium répondant à la formule (I)

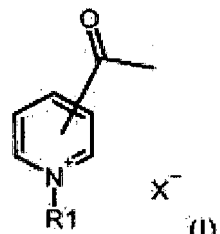

(I),

dans laquelle
R1 représente un groupe alkyle en $C_1$-$C_6$, un groupe alcényle en $C_2$-$C_6$, un groupe hydroxyalkyle en $C_2$-$C_6$, un groupe alcoxy(en $C_1$-$C_6$)alkyle en $C_2$-$C_6$, un groupe carboxyalkyle en $C_2$-$C_6$, un groupe arylalkyle en $C_1$-$C_6$, un groupe hétéroarylalkyle en $C_1$-$C_6$, un groupe aryle ou un groupe hétéroaryle ; et
$X^-$ représente un anion physiologiquement acceptable.

**8.** Unité de conditionnement à plusieurs composants (kit-of-parts) comprenant au moins trois composants confectionnés séparés les uns des autres, **caractérisée en ce que**

i) le premier composant représente une préparation (A) pour la teinture contenant, dans un support cosmétique, au moins un précurseur de colorant d'oxydation ;
ii) le deuxième composant représente une préparation d'agent d'oxydation (B) contenant, dans un support cosmétique, au moins un agent d'oxydation ; et
iii) le troisième composant représente un agent de traitement ultérieur (C), qui contient, dans un support cosmétique, au moins un dérivé d'acétylpyridinium répondant à la formule (I)

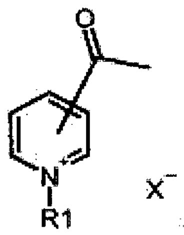
(I),

dans laquelle

R1 représente un groupe alkyle en $C_1$-$C_6$, un groupe alcényle en $C_2$-$C_6$, un groupe hydroxyalkyle en $C_2$-$C_6$, un groupe alcoxy(en $C_1$-$C_6$)alkyle en $C_2$-$C_6$, un groupe carboxyalkyle en $C_2$-$C_6$, un groupe arylalkyle en $C_1$-$C_6$, un groupe hétéroarylalkyle en $C_1$-$C_6$, un groupe aryle ou un groupe hétéroaryle ; et

$X^-$ représente un anion physiologiquement acceptable.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- DE 19745356
- DE 102007047685


### In der Beschreibung aufgeführte Nicht-Patentliteratur


- **B. KH. SCHRADER.** Grundlagen und Rezepturen der Kosmetika. Hüthig Buch Verlag, 1989